(19) Europäisches Patentamt
European Patent Office
Office européen des brevets

(11) **EP 3 501 453 A1**

(12) **EUROPÄISCHE PATENTANMELDUNG**

(43) Veröffentlichungstag:
**26.06.2019 Patentblatt 2019/26**

(51) Int Cl.:
*A61F 2/24* *(2006.01)*          *A61F 2/95* *(2013.01)*

(21) Anmeldenummer: **17209494.8**

(22) Anmeldetag: **21.12.2017**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA MD TN**

(71) Anmelder: **Biotronik AG**
**8180 Bülach (CH)**

(72) Erfinder:
• **Pfenniger, Alois**
  **2502 Biel (CH)**
• **Bitzer, Andreas**
  **8032 Zürich (CH)**

(74) Vertreter: **Galander, Marcus**
  **Biotronik Corporate Services SE**
  **Corporate Intellectual Property**
  **Sieversufer 7 - 9**
  **12359 Berlin (DE)**

(54) **KATHETEREINRICHTUNG MIT RINGSTRUKTUR ZUR ERLEICHTERUNG DES WIEDEREINFÜHRENS (RESHEATHING) EINER TEILWEISE FREIGESETZTEN HERZKLAPPENPROTHESE**

(57)    Die Erfindung betrifft eine Kathetereinrichtung zum Implantieren einer Herzklappenprothese (2), mit: einer Herzklappenprothese (2), die eine Herzklappe aufweist und ein selbst-expandierbares Stentgerüst (3), das die Herzklappe trägt, einem Außenschaft (6), der an einem distalen Ende eine Kapsel (4) aufweist, die die Herzklappenprothese (2) umgibt, wobei die Kapsel (4) und die Herzklappenprothese (2) relativ zueinander verschiebbar sind, so dass die Herzklappenprothese abschnittsweise freisetzbar ist, wobei ein freigesetzter Abschnitt (T) des Stentgerüsts (3) sich selbständig expandiert, sowie in die Kapsel (4) wieder einführbar ist. Erfindungsgemäß ist vorgesehen, dass die Kathetereinrichtung (1) zum Schutz der Herzklappenprothese (2) beim Wiedereinführen der Herzklappenprothese (2) in die Kapsel (4) eine zusätzliche Ringstruktur (5) aufweist, die in distaler Richtung (R') über die Kapsel (4) hinaus verschiebbar ist, so dass ein bereits freigesetzter, expandierter Abschnitt der Herzklappenprothese (2) mittels der Ringstruktur (5) kontaktierbar ist.

**FIG. 2A**

FIG. 2B

FIG. 2C

FIG. 2D

**Beschreibung**

Die Erfindung betrifft eine Kathetereinrichtung gemäß dem Oberbegriff des Anspruchs 1. Die Erfindung wird am Beispiel einer Kathetereinrichtung zur Implantation einer selbstexpandierenden Herzklappenprothese beschrieben, ist aber im Prinzip für jede Kathetereinrichtung mit einem selbstexpandierenden Implantat geeignet, welches nach teilweiser Freisetzung wieder auf seinen ursprünglichen Durchmesser komprimiert werden soll.

Derartige Kathetereinrichtungen dienen z.B. zur Implantation von Herzklappenprothesen, z.B. bei einer Transkatheter-Aortenklappen-Implantation.

Hierbei verwendete Herzklappenprothesen weisen in der Regel eine Herzklappe auf, die z.B. aus einem biologischen Material gefertigt ist und an einem Stentgerüst festgelegt ist, dass als Träger der Herzklappe sowie insbesondere zur späteren Verankerung der Herzklappe im Herzen dient. Die verwendeten Stentgerüste können insbesondere selbst-expandierend ausgebildet sein.

Bei einer solchen selbständigen Expansion des Stentgerüstes erfolgt vor allem eine Aufweitung des Stentgerüstdurchmessers bzw. des Durchmessers der Herzklappenprothese in radialer Richtung, d.h., in einer senkrecht zur Längsachse bzw. axialen Richtung der Kathetereinrichtung orientierten Ebene.

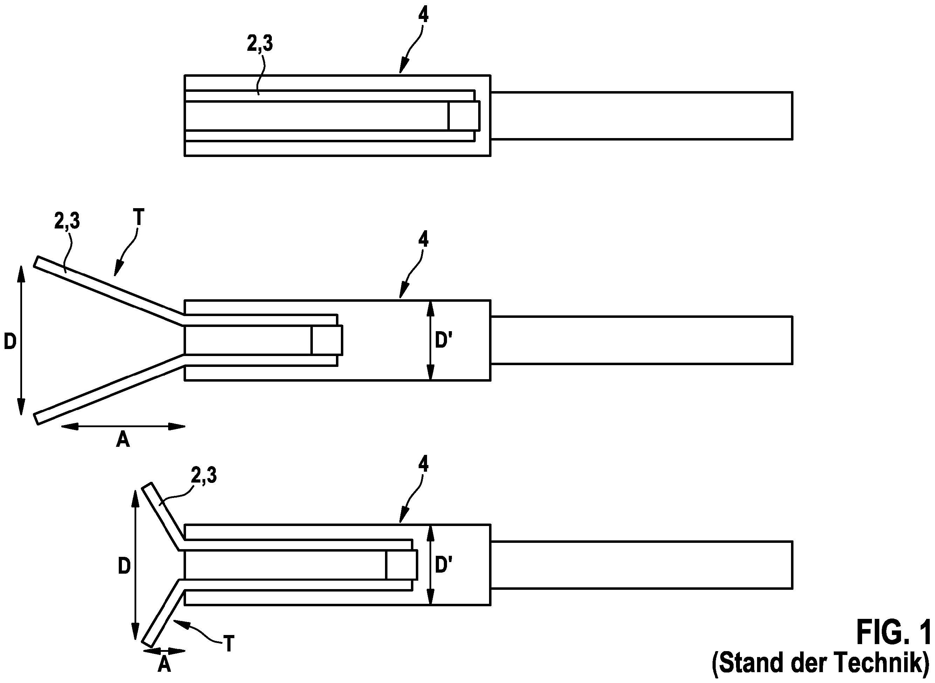

Wie in der Figur 1 schematisch angedeutet, ist dies insofern problematisch, als der Durchmesser D des bereits freigesetzten Abschnittes der Herzklappenprothese bzw. des Stentgerüstes den Durchmesser D' der Kapsel 4 in radialer Richtung deutlich überschreiten kann. Beim Wiedereinführen (Resheating) der Herzklappenprothese 2 in die Kapsel 4 muss der Durchmesser des bereits entfalteten Teiles T wiederum im Wesentlichen auf den ursprünglichen Durchmesser reduziert werden. Hierdurch wird beim Wiedereinführen die Herzklappenprothese 2 bzw. das zugehörige Stentgerüst 3 in eine regenschirmartige Form deformiert, bei der der Durchmesser D des Stentgerüstes bzw. der Herzklappenprothese in radialer Richtung auf kurzer axialer Distanz A auf den Kapseldurchmesser am distalen Rand der Kapsel 4 abfällt. Ein Wiedereinführen der Herzklappenprothese in die Kapsel wird zum Beispiel notwendig, wenn die Positionierung des teilweise freigesetzten Implantats korrigiert werden muss oder die Implantation aus anderen Gründen abgebrochen und die Herzklappenprothese aus dem Körper wieder entfernt werden muss.

Hierbei übt die Kapsel mit ihrem distalen Rand, der eine Öffnung der Kapsel umgibt, über die die Herzklappenprothese wieder in die Kapsel einzuziehen ist, entsprechend große axiale Kräfte (d.h. in Richtung der Längsachse der Kathetereinrichtung wirkende Kräfte) auf das Stentgerüst bzw. die Herzklappenprothese aus, die das Stentgerüst/Herzklappenprothese und die Kapsel belasten und das Wiedereinführen (sogenanntes resheathing) erschweren. Dabei kann es auch vorkommen, dass sich die Herzklappenprothese nicht mehr vollständig in die Kapsel zurückziehen lässt, wodurch der nicht von der Kapsel bedeckte Teil der Herzklappenprothese radial nach außen absteht. In einem derartigen Zustand kann die Herzklappenprothese weder implantiert noch gefahrlos aus dem Körper entfernt werden.

Der vorliegenden Erfindung liegt daher die Aufgabe zugrunde, eine Kathetereinrichtung bereitzustellen, die hinsichtlich der vorgenannten Problematik verbessert ist.

Diese Aufgabe wird durch eine Kathetereinrichtung mit den Merkmalen des Anspruchs 1 gelöst. Vorteilhafte Ausgestaltungen der Erfindung sind in den Unteransprüchen angegeben und werden nachfolgend beschrieben.

Gemäß Anspruch 1 wird eine Kathetereinrichtung zum Implantieren einer Herzklappenprothese offenbart, mit:

- einer Herzklappenprothese, die eine Herzklappe und ein selbst-expandierbares Stentgerüst aufweist, das die Herzklappe trägt,
- einem Außenschaft, der an einem distalen Ende eine Kapsel aufweist, die die Herzklappenprothese umgibt, wobei die Kapsel und die Herzklappenprothese relativ zueinander verschiebbar sind, so dass die Herzklappenprothese abschnittsweise freisetzbar ist, wobei ein freigesetzter Abschnitt des Stentgerüsts sich selbständig expandiert, sowie in die Kapsel (insbesondere vollständig) wieder einführbar ist.

Erfindungsgemäß ist nun vorgesehen, dass die Kathetereinrichtung zum Schutz der Herzklappenprothese (bzw. des Stentgerüstes) beim Wiedereinführen (sogenanntes resheathing) der Herzklappenprothese in die Kapsel und/oder zum Komprimieren der Herzklappenprothese in radialer Richtung eine (insbesondere zusätzliche) Ringstruktur aufweist, die über einen bereits freigesetzten, expandierter Abschnitt der Herzklappenprothese (insbesondere des Stentgerüstes) positionierbar ist (so dass die Ringstruktur diesen Abschnitt umgreift), wobei die Ringstruktur insbesondere in distaler Richtung verschiebbar ist, so dass der bereits freigesetzte, expandierte Abschnitt der Herzklappenprothese (insbesondere des Stentgerüstes) mittels der Ringstruktur kontaktierbar und/oder komprimierbar ist und insbesondere beim Wiedereinführen der Herzklappenprothese in die Kapsel mittels der Ringstruktur führbar ist. Die Ringstruktur kann dabei radial über der Kapsel angeordnet sein (die Ringstruktur umgibt die Kapsel).

Gemäß einer Ausführungsform der Erfindung ist vorgesehen, dass die Ringstruktur in distaler Richtung über die Kapsel hinaus verschiebbar ist, so dass ein bereits freigesetzter, expandierter Abschnitt der Herzklappenprothese (insbesondere des Stentgerüstes) mittels der Ringstruktur kontaktierbar ist.

Im Sinne der vorliegenden Erfindung bedeutet distal, dass eine entsprechende distale Komponente, ein distaler Abschnitt oder ein distales Ende in der axialen Richtung des Außenschaftes, entlang der sich der Außenschaft bzw. die Längsachse des Außenschaftes erstreckt, weiter von einem Handgriff bzw. einem Bediener (Arzt) der Kathetereinrichtung entfernt ist, als eine proximale Komponente, ein proximaler Abschnitt bzw. ein proximales Ende. Entsprechend weist die distale Richtung entlang der Kathetereinrichtung von proximal nach distal und die proximale Richtung entsprechend entlang der Kathetereinrichtung von distal nach proximal. Die radiale Richtung steht jeweils senkrecht auf der besagten axialen Richtung.

Das Stentgerüst ist vorzugsweise selbst-expandierend ausgestaltet. D.h., es weist ein Material auf oder es besteht aus einem Material, das ein Komprimieren des Stentgerüstes in radialer Richtung auf einen reduzierten Durchmesser zulässt, wobei eine selbsttätige Entfaltung bzw. Expansion des Stentgerüstes in radialer Richtung auf den ursprünglichen Durchmesser erfolgt, sobald das Stentgerüst keiner äußeren Beschränkung seines Durchmessers mehr unterworfen ist (z.B. wenn das Stentgerüst aus seiner das Stentgerüst umgebenden Kapsel freigesetzt wird). Hierbei wird auch die Herzklappe, die am Stentgerüst festgelegt ist und bei der es sich insbesondere um eine biologische Herzklappe handeln kann, entfaltet bzw. aufgespannt. Geeignete Materialien, die diese Eigenschaften aufweisen sind im Stand der Technik hinlänglich bekannte Form-Gedächtnis Materialien (z.B. Nickel-Titan-Legierungen wie z.B. Nitinol).

In einer bevorzugten Ausgestaltung der Erfindung ist die Herzklappenprothese im distalen Abschnitt eines Katheterschaftes angeordnet, welcher gegenüber dem Außenschaft verschieblich ist. Die Herzklappenprothese ist dabei mit diesem Katherschaft (im Folgenden auch Innenschaft genannt) bis zu ihrer völligen Freisetzung lösbar verbunden.

Gemäß einer bevorzugten Ausführungsform der Erfindung ist vorgesehen, dass die Ringstruktur dazu ausgebildet ist, einen Durchmesser des Stentgerüstes am Ort der Ringstruktur zu begrenzen, so dass entsprechend eine axiale Kraft, die beim Wiedereinführen der Herzklappenprothese in die Kapsel auf die Kapsel selbst ausgeübt wird, begrenzt wird. Die Ringstruktur verringert beim Wiedereinführen den Durchmesser des Stents und erleichtert so das Wiedereinführen der Herzklappenprothese in die Kapsel.

Die Ringstruktur ist gemäß einer Ausführungsform der Erfindung insbesondere derart arretierbar ausgestaltet, dass sie beim Wiedereinführen der Herzklappenprothese in die Kapsel ihre räumliche Lage bezüglich der Kapsel beibehält. Die Ringstruktur kann also beim Wiedereinführen der Herzklappenprothese in die Kapsel axiale Kräfte aufnehmen und ist insbesondere dazu ausgebildet, den Durchmesser des Stentgerüstes beim Wiedereinführen der Herzklappenprothese in die Kapsel bereits außerhalb der Kapsel zu begrenzen, so dass eine übermäßige (regenschirmartige) Deformierung des Stentgerüstes bzw. der Herzklappenprothese beim Wiedereinführen verhindert wird.

Weiterhin ist gemäß einer Ausführungsform der Erfindung vorgesehen, dass die Kathetereinrichtung zum Tragen der Ringstruktur eine Mehrzahl an Stäben aufweist, die jeweils über einen ersten Endabschnitt mit der Ringstruktur verbunden sind. Die Stäbe müssen dabei nicht notwendigerweise einstückig ausgebildet ein, sondern können sich aus einzelnen Abschnitten zusammensetzen. So können die Stäbe z.B. in axialer Richtung komprimierbare Abschnitte aufweisen, z.B. in Form einer Feder (siehe auch unten).

Weiterhin ist gemäß einer Ausführungsform der Erfindung vorgesehen, dass die Stäbe dazu konfiguriert sind, die Ringstruktur in axialer bzw. distaler Richtung über die Kapsel hinaus zu verschieben, so dass die Ringstruktur die teilweise entfaltete Herzklappenprothese (insbesondere das Stentgerüst) in der oben beschriebenen Weise kontaktieren kann.

Weiterhin ist gemäß einer Ausführungsform der Erfindung vorgesehen, dass die Kathetereinrichtung einen äußeren Stabilisierungsschaft zum Stabilisieren des Außenschaftes aufweist, wobei der Außenschaft in einem vom Stabilisierungsschaft umgebenden Lumen des Stabilisierungsschaftes angeordnet ist, und wobei der Außenschaft und der Stabilisierungsschaft relativ zueinander verschiebbar sind.

Hinsichtlich der Stäbe ist weiterhin in einer Ausführungsform der Erfindung vorgesehen, dass diese jeweils über einen zweiten Endabschnitt, der dem jeweiligen ersten Endabschnitt in axialer Richtung gegenüberliegt, an einem distalen Ende des Stabilisierungsschaftes festgelegt sind. Die Ringstruktur lässt sich daher über die Stäbe sowie den Stabilisierungsschaft in Richtung auf die am Außenschaft festgelegte Kapsel bzw. über die Kapsel hinaus bewegen.

In einer Ausgestaltung der Erfindung, wo die Stäbe am distalen Ende des Stabilisatorschaftes festgelegt sind, ist der Stabilisatorschaft gegenüber dem Außenschaft und gegenüber der Herzklappenprothese (und damit dem Innenschaft) axial verschieblich. Hierbei ist es unerheblich, ob zwischen den Außenschaft, welcher die Herzklappenprothese im radial komprimierten Zustand hält, und dem Stabilisatorschaft noch ein oder mehrere weitere Katheterschäfte angeordnet sind. Ebenfalls können radial außerhalb des Stabilisatorschaftes noch ein oder mehrere weitere Katheterschäfte angeordnet sein. Entscheidend ist in dieser Ausgestaltung die axiale Verschiebbarkeit zwischen Stabilisatorschaft und Außensowie Innenschaft. Es kommt ebenfalls nicht darauf an, welcher Katheterschaft in welche Richtung beweglich ist, entscheidend ist allein ihre relative Beweglichkeit zueinander. Bevorzugt kann der Außenschaft nach proximal zurück gezogen und der Stabilisatorschaft nach distal vorgeschoben werden, während der Innenschaft nicht bewegt wird.

Die Kathetereinrichtung weist weiterhin gemäß einer Ausführungsform der Erfindung einen Innenschaft auf, der in einem Lumen des Außenschaftes angeordnet ist, wobei an einem distalen Ende des Innenschaftes ein Träger vorgesehen ist, der die Herzklappenprothese trägt. Vorzugsweise sind der Außen- und der Innenschaft gemäß einer Ausführungsform relativ zueinander verschiebbar, so dass der Träger zum Freisetzen bzw. zum abschnittsweise Freisetzen der Herzklappenprothese aus der Kapsel herausführbar ist. Am Träger kann eine Befestigungsstruktur vorgesehen sein, die mit einem Teil des Stentgerüstes in Eingriff steht, der z.B. an einem Abschnitt des Stentgerüstes vorgesehen ist, der als letztes freigesetzt wird, so dass eine lediglich teilweise bzw. abschnittsweise freigesetzte Herzklappenprothese wieder in die Kapsel einführbar ist (durch eine entsprechende Relativbewegung des Trägers und der Kapsel).

Weiterhin kann in einer Ausführungsform der Innenschaft ein Lumen aufweisen, in dem ein Führungsdraht verläuft, so dass der Innenschaft (mit Träger), der Außenschaft (mit Kapsel) und der Stabilisierungsschaft durch den Führungsdraht führbar sind.

Weiterhin ist gemäß einer Ausführungsform der Erfindung vorgesehen, dass die zweiten Endabschnitte der Stäbe über ein Gelenk mit dem distalen Ende des Stabilisierungsschaftes verbunden sind. Insbesondere bei einer Transkatheter-Aortenklappen-Implantation (kurz TAVI) ist die Kathetereinrichtung deutlichen Biegungsdeformationen ausgesetzt (insbesondere im Aortenbogen). Mittels des Gelenkes können die unterschiedlichen Längen der gebogenen Stäbe bezüglich der Mittellinie der Kathetereinrichtung mit Vorteil kompensiert werden.

Hinsichtlich des besagten Gelenkes ist gemäß einer Ausführungsform der Erfindung vorgesehen, dass das Gelenk einen ersten Gelenkring, einen zweiten Gelenkring sowie einen dritten Gelenkring aufweist, wobei der zweite Gelenkring zwischen dem ersten und dem dritten Gelenkring angeordnet ist, und wobei die drei Gelenkringe den Außenschaft in Umfangsrichtung des Außenschaftes umfassen, wobei der erste Gelenkring um eine erste Achse kippbar mit dem zweiten Gelenkring verbunden ist, und wobei der zweite Gelenkring um eine zweite Achse kippbar mit dem dritten Gelenkring verbunden ist, wobei die beiden Achsen orthogonal zueinander verlaufen. Weiterhin ist zur Verbindung dieser Gelenkstruktur mit dem Stabilisierungsschaft bzw. mit den Stäben vorgesehen, dass der erste Gelenkring mit den zweiten Endabschnitten der Stäbe starr verbunden ist, und dass der dritte Gelenkring mit dem distalen Ende des Stabilisierungsschaftes starr verbunden ist.

Die vorangehend geschilderte Ausgestaltung mit einem Gelenk bestehend aus 3 Gelenkringen gewährleistet eine Kippbarkeit in alle Raumrichtungen durch die orthogonale Anordnung der beiden Achsen. Bei einer Ausgestaltung, wo von vornherein nur die Kippbarkeit um eine vorgegebene Achse erforderlich ist, sind 2 Gelenkringe ausreichend. Analog kann es für eine feinere Einstellung der Kippbarkeiten der Achse vorteilhaft sein, mehr als 3 Gelenkringe zu verwenden. Die Ausgestaltung aus 3 Gelenkringen stellt jedoch das Optimum in Bezug auf Aufwand bei freier Beweglichkeit der Anordnung dar.

Grundsätzlich sind für die besagte Ringstruktur verschiedene Formen denkbar. So kann die Ringstruktur z.B. einstückig ausgebildet sein oder sie kann aus mehreren Elementen zusammengesetzt sein.

Diesbezüglich ist gemäß einer Ausführungsform der Erfindung vorgesehen, dass die Ringstruktur ein durchgehender, in sich geschlossener und selbstexpandierender Ring ist.

Weiterhin ist gemäß einer alternativen Ausführungsform der Erfindung vorgesehen, dass die Ringstruktur durch eine Mehrzahl an Ringelementen gebildet ist, wobei die Ringelemente zueinander bewegbar sind, derart, dass die Ringstruktur aus einer ersten Konfiguration in eine zweite Konfiguration bewegbar ist, wobei die Ringstruktur in der zweiten Konfiguration einen größeren Durchmesser aufweist als in der ersten Konfiguration.

Die Ringstruktur kann also mit Vorteil in der ersten Konfiguration mittels der Kathetereinrichtung zum Implantationsort transportiert werden, wobei dann gegebenenfalls eine Entfaltung/Expansion der Ringstruktur die zweite Konfiguration erfolgen kann, in der die Ringstruktur einen größeren Durchmesser aufweist und die teilweise freigesetzte Herzklappenprothese beim Wiedereinführen in die Kapsel geeignet abstützen kann (siehe oben).

Bei einer Ringstruktur in Form eines in sich geschlossenen, einstückigen Ringes können die Stäbe mit ihren ersten Endabschnitten geeignet an dem Ring festgelegt sein (z.B. über je eine Lötverbindung).

Für den Fall, dass sich die Ringstruktur aus separaten Ringelementen zusammensetzt, ist gemäß einer Ausführungsform bevorzugt vorgesehen, dass jeder Stab über seinen ersten Endabschnitt mit einem zugordneten Ringelement verbunden ist. Insbesondere können jeweils zwei parallele Stäbe mit dem gleichen Ringelement verbunden sein, z.B. wenn das Ringelement buchtartig ausgebildet ist und entsprechend zwei Enden aufweist (siehe unten).

Weiterhin ist gemäß einer Ausführungsform der Erfindung vorgesehen, dass je zwei benachbarte Ringelemente ineinandergreifen, so dass die Ringstruktur durch eine Kette der Ringelemente gebildet ist, wobei insbesondere die Ringelemente bzw. Kettenglieder in Umfangsrichtung der Ringstruktur zusammenschiebbar angeordnet sind, so dass der Durchmesser der Ringstruktur entsprechen einstellbar ist. In der ersten Konfiguration liegen die Ringelemente in Umfangsrichtung des Außenschaftes dann enger zusammen als in der zweiten Konfiguration.

Die verketteten Ringelemente können insbesondere jeweils buchtförmig ausgebildet sein, d.h. sie weisen jeweils einen gekrümmten Abschnitt sowie zwei einander gegenüberliegende Enden auf, die in Umfangsrichtung des Außenschaftes zueinander beabstandet sind. An diese Enden können sich dann die Stäbe anschließen, wobei die Stäbe dann jeweils integral mit den Ringelementen bzw. deren Enden verbunden sein können.

Weiterhin ist gemäß einer Ausführungsform der Erfindung vorgesehen, dass die einzelnen Ringelemente nicht miteinander in Eingriff stehen, sondern unter Ausbildung der Ringstruktur nebeneinander angeordnet sind. Diesbezüglich ist gemäß einer Ausführungsform vorgesehen, dass je zwei benachbarte Ringelemente in der ersten Konfiguration axial zueinander versetzt angeordnet sind, so dass die Ringstruktur erste Ringelemente aufweist, die in der ersten Konfiguration der Ringstruktur in distaler Richtung vor zweiten Ringelementen der Ringstruktur angeordnet sind. Weiterhin ist gemäß einer Ausführungsform vorgesehen, dass in der zweiten Konfiguration sämtliche Ringelemente in der Umfangsrichtung nebeneinander angeordnet sind und sich insbesondere in axialer Richtung bzw. distaler Richtung auf der gleichen Position bzw. Höhe befinden.

Hierbei kann das jeweilige Ringelement wiederum buchtförmig, insbesondere geschlossen buchtförmig, ausgebildet sein. D.h., das jeweilige Ringelement weist wiederum einen gekrümmten Abschnitt auf sowie zwei einander gegenüberliegende Enden, die insbesondere aneinander anliegen.

Bei diesen Ringelementen ist also bevorzugt vorgesehen, dass eine Breite des jeweiligen Ringelementes bzw. der jeweiligen Bucht in Umfangsrichtung der Ringstruktur abseits der Enden des Ringelementes größer ist als die Breite des Ringelementes bzw. der jeweiligen Bucht in Umfangsrichtung an den Enden des Ringelementes. Hierdurch kann bei entsprechend axial versetzter Anordnung von benachbarten Ringelementen der Durchmesser der Ringstruktur insgesamt verringert werden, da sich ein Ringelement im Bereich der Enden des benachbarten (ersten) Ringelementes an das benachbarte Ringelement (bzw. an dessen Stäbe) anschmiegen kann.

Weiterhin ist gemäß einer Ausführungsform der Erfindung vorgesehen, dass die mit den ersten Ringelementen verbundenen Stäbe einen in axialer Richtung elastisch komprimierbaren Abschnitt aufweisen, der z.B. durch eine Feder gebildet sein kann, so dass, wenn die Ringstruktur in distaler Richtung über die Kapsel hinaus verschoben wird, die ersten Ringelemente bei einem Kontakt mit der abschnittsweise freigesetzten Herzklappenprothese gegen eine rückstellende Kraft des jeweiligen elastisch komprimierbaren Abschnittes zurückgeschoben werden, bis alle Ringelemente eine gleiche Position in axialer Richtung aufweisen. Somit erfolgt in dieser Ausführungsform der Erfindung eine automatische Positionierung der Ringelemente zur Herstellung der zweiten Konfiguration der Ringstruktur durch Kontakt mit der abzustützen Herzklappenprothese.

Weiterhin kann gemäß einer Ausführungsform der Erfindung vorgesehen sein, dass die Stäbe von einer äußeren, den Außenschaft umgebend Hülle umgeben sind oder an einer äußeren, den Außenschaft umgebenden Hülle festgelegt sind. Insbesondere kann die äußere Hülle elastisch ausgebildet sein und kann insbesondere bis zu einem vordefinierten Durchmesser radial gedehnt werden. Auf diese Weise kann die Hülle einen maximalen Durchmesser der Ringstruktur begrenzen. Alternativ kann die Begrenzung des maximalen Durchmessers der Ringstruktur auch über Drähte, Bänder oder ähnliches erfolgen.

Die Stäbe können des Weiteren insbesondere an der Hülle festgelegt sein, indem sie die Hülle an mehreren Stellen durchstoßen, so dass sie alternierend unterhalb und oberhalb der Hülle verlaufen.

Die besagte Hülle kann z.B. aus einem Kunststofffasergewebe, wie beispielsweise Dacron® Gewebe, oder einem anderen geeigneten Material, insbesondere Gewebe, bestehen. Grundsätzlich ist hier jedes Gewebe geeignet, welches eine begrenzte Dehnbarkeit in Umfangsrichtung aufweist, wobei unter Gewebe im Rahmen der Anmeldung ein Verbund aus einzelnen Fasern verstanden wird.

Gemäß einer weiteren Ausführungsform der Erfindung ist vorgesehen, dass mittels der Ringstruktur (alternativ oder ergänzend) die Herzklappenprothese, insbesondere das Stentgerüst, aktiv im Durchmesser, d.h., in radialer Richtung, komprimierbar ist.

Es ist also bei dieser Ausführungsform vorgesehen, dass die Kathetereinrichtung eine Ringstruktur aufweist, die in distaler Richtung verschiebbar ist, und zwar insbesondere über einen bereits freigesetzten, expandierten Abschnitt der Herzklappenprothese, so dass diese mittels der Ringstruktur komprimierbar ist.

Die Ringstruktur weist hierzu gemäß einer Ausführungsform der Erfindung einen verringerbaren Durchmesser auf, so dass die Ringstruktur um die Herzklappenprothese zusammenziehbar ist und dabei eine Kraft in radialer Richtung auf die Herzklappenprothese ausüben kann, die die Herzklappenprothese, insbesondere das Stentgerüst, in radialer Richtung komprimiert, so dass die Herzklappenprothese wieder in die Kapsel zurückbewegbar ist (Resheathing).

Gemäß einer Ausführungsform ist vorgesehen, dass die Ringstruktur eine Mehrzahl an Stäben aufweist sowie ein längserstrecktes flexibles Element. Die Stäbe weisen jeweils ein erstes Ende sowie eine am jeweiligen ersten Ende vorgesehene Durchgangsöffnung auf, wobei das längserstreckte flexible Element durch die Durchgangsöffnungen geführt ist.

Bei dem Element kann es sich um ein biegeschlaffes Element handeln. Unter dem längserstreckten flexiblen Element, welches insbesondere biegeschlaff ist, wird im Rahmen dieser Anmeldung ein Element verstanden, welches sehr einfach in jede Raumrichtung biegbar oder bewegbar ist, jedoch nicht entlang seiner Längsachse gedehnt oder gestreckt werden kann. Bei einem derartigen biegeschlaffen flexiblen Element kann es sich beispielsweise um einen Draht, einen Faden, oder ein Seil handeln. Das Element bildet insbesondere einen in sich geschlossenen Ring, so dass ein Rotieren der Stäbe um die Längsachse der Stäbe dazu führt, dass sich das flexible Element um den jeweiligen Stab wickelt, so dass die Ringstruktur im Durchmesser verringert wird, sich dabei um die teilweise expandierte Herzklappenprothese zusammenzieht und diese in radialer Richtung zum Wiedereinführen in die Kapsel komprimiert. Die Stäbe können insbesondere mittels des Stabilisierungsschaft in distaler Richtung verschiebbar sein, so dass die Ringstruktur mittels des Stabilisierungsschaft über die teilweise expandierte Herzklappenprothese schiebbar und dort im Durchmesser verringerbar ist. Die Stäbe können weiterhin über ihre zweiten Enden, die den ersten Enden gegenüberliegen, mit einem Mechanismus gekoppelt sein, mittels dem die Stäbe um die jeweilige Längsachse rotierbar sind.

Gemäß einer Ausführungsform können die ersten Enden kugelförmig ausgebildet sein.

Weiterhin kann die Ringstruktur ein ringförmiges Element aufweisen, wobei die ersten Enden der Stäbe jeweils in eine zugeordnete Ausnehmung des ringförmigen Elementes eingreifen und dort um die Längsachse des jeweiligen Stabes rotierbar gelagert sind. Die jeweilige Ausnehmung bildet dabei ein Drehlager für das erste Ende des jeweiligen Stabes.

Die Ausnehmungen bzw. Gleitlager können jeweils in einem starren Abschnitt des ringförmigen Elementes angeordnet sein, wobei je zwei in Umfangsrichtung benachbarte starre Abschnitte durch einen Zwischenabschnitt, insbesondere einen elastisch deformierbaren Zwischenabschnitt, miteinander verbunden sind. Durch rotieren der Stäbe können die elastisch deformierbaren Abschnitte in Umfangsrichtung des ringförmigen Elementes zusammengedrückt werden, wobei der Durchmesser des ringförmigen Elementes verringert wird.

Gemäß einer weiteren Ausführungsform ist vorgesehen, dass die Ringstruktur durch die Kapsel selbst gebildet wird.

Hierbei weist die Kapsel gemäß einer Ausführungsform zumindest ein, bevorzugt zumindest drei Wandelemente auf, wobei in Umfangsrichtung der Kapsel benachbarte Wandelemente einander überlappen, und wobei jedes Wandelement ein Lager für einen ersten Endabschnitt eines Stabes aufweist, wobei jeder Stab in seinem Lager um seine Längsachse rotierbar ist, und wobei jeder Stab eine Durchgangsöffnung aufweist durch die ein längserstrecktes flexible Element geführt ist.

Bei dem längserstreckten, flexiblen Element kann es sich wiederum um ein biegeschlaffes Element (analog oben) handeln. Weiterhin kann es sich bei dem Element um einen Draht, einen Faden, oder ein Seil handeln. Das Element bildet insbesondere einen in sich geschlossenen Ring, so dass ein Rotieren der Stäbe um die Längsachse der Stäbe dazu führt, dass sich das flexible Element um den jeweiligen Stab wickelt, so dass je zwei einander überlappende Wandelemente in Umfangsrichtung übereinander geschoben werden, wobei die die Wandelemente aufweisende Ringstruktur im Durchmesser verringert wird und sich dabei um die teilweise expandierte Herzklappenprothese zusammenzieht und diese in radialer Richtung zum Wiedereinführen in die Kapsel komprimiert. Die Stäbe können wiederum über ihre zweiten Endabschnitte, die den ersten Endabschnitten gegenüberliegen, mit einem Mechanismus gekoppelt sein, mittels dem die Stäbe um die jeweilige Längsachse rotierbar sind. Ausgestaltungen mit mehr als 3 Wandelementen funktionieren analog zur vorangehenden Schilderung, Ausgestaltungen mit einem oder zwei Wandelementen ähnlich. Wird beispielsweise nur ein Wandelement verwendet, überlappt dieses mit sich selbst. D.h. der Umfang dieses Wandelementes ist größer als es für den Durchmesser erforderlich ist.

Für Wandelemente sind Materialien vorteilhaft, wie sie auch für Katheterschäfte (beispielsweise den Außenschaft) verwendet werden. Dies sind Kunststoffe wie Polymere, insbesondere Polyethylene wie HDPE.

Gemäß einer Ausführungsform ist vorgesehen, dass die Ringstruktur wiederum eine Mehrzahl an Stäben aufweist sowie ein längserstrecktes flexibles Element. Die Stäbe weisen jeweils ein erstes Ende auf an dem der jeweilige Stab eine Durchgangsöffnung aufweist, wobei das längserstreckte flexible Element durch die Durchgangsöffnungen geführt ist. Die ersten Enden können jeweils kugelförmig ausgebildet sein.

Weiterhin ist jedes erste Ende mit einem schraubenlinienförmig gekrümmten Verbindungsstab verbunden (insbesondere einstückig), wobei der jeweilige Verbindungstab einen abgewinkelten Endabschnitt aufweist, der in einem kugelförmigen Ende aufweist, das eine Durchgangsöffnung aufweist, wobei in der jeweiligen Durchgangsöffnung ein benachbarter Verbindungstab gleitend gelagert ist.

Bei dem längserstreckten flexiblen Element kann es sich wiederum um ein biegeschlaffes Element handeln. Weiterhin kann es sich bei dem Element um einen Draht, einen Faden, oder ein Seil handeln. Das Element bildet insbesondere einen in sich geschlossenen Ring, so dass ein Rotieren der Stäbe um die Längsachse der Stäbe dazu führt, dass sich das flexible Element um den jeweiligen Stab wickelt, so dass die Ringstruktur im Durchmesser verringert wird, wobei sich die Stäbe und die Verbindungstäbe um die teilweise expandierte Herzklappenprothese zusammenziehen und diese in radialer Richtung zum Wiedereinführen in die Kapsel komprimieren. Die Stäbe können weiterhin über ihre zweiten Enden, die den ersten Enden gegenüberliegen, mit einem Mechanismus gekoppelt sein, mittels dem die Stäbe um die jeweilige Längsachse rotierbar sind.

Vorteilhafterweise kann die Ringstruktur und die zugehörigen optionalen Stäbe, Gelenkstrukturen oder Elemente mit einer zusätzlichen dünnen Hülle umgeben sein. Diese zusätzliche optionale dünne Hülle oder Folie schützt das Gewebe am Implantationsort vor einem direkten Kontakt mit der Ringstruktur und deren Zubehör.

Die erfindungsgemäße Lösung erlaubt in vorteilhafter Weise ein resheathing der Herzklappenprothese, wobei die üblicherweise auftretende, regenschirmartige Verformung des Stentgerüstes bzw. der Herzklappenprothese verhindert werden kann. Durch die Erfindung werden die axial auf das Stentgerüst beim Wiedereinführen in die Kapsel wirkenden Kräfte reduziert, so dass die Herzklappenprothese und die Kapsel entsprechend geschont wird, was sich auf die Langzeitperformance der Herzklappenprothese positiv auswirkt.

Weiterhin kann die erfindungsgemäße Lösung auf alle erdenklichen Katheterformen übertragen werden. Die erfindungsgemäße Einrichtung kann somit auch als Zusatzwerkzeug für ein bereits bestehendes Kathetersystem bereitgestellt werden.

Die vorstehend beschriebenen Ausführungsformen, die ein Komprimieren der teilweise expandierten Herzklappenprothese mittels der Ringstruktur erlauben weisen mehrere Vorteile auf. Der in der aufsteigenden Aorta zur Verfügung stehende Raum wird durch die expandierbare Geometrie nutzbar. Hierdurch können das Kathetersystem betreffende Einschränkungen während des Resheathing reduziert werden. Der Resheathing-Mechanismus kann als Zusatzwerkzeug ausgestaltet sein, so dass er auf eine Vielzahl von (bereits existierenden) Kathetersystemen anwendbar ist. Weiterhin werden in einigen Ausgestaltungen axiale Kräfte reduziert, da die Aktuierung des Mechanismus auf Torsion beruht. Hierdurch wird das Risiko der Ausbeulung einer länglichen Struktur, die in axialer Richtung belastet ist, vermieden. Ferner kann die Herzklappenprothese, insbesondere das Stentgerüst, durch radiale Kräfte komprimiert werden, bevor es in die Kapsel zurückbewegt wird. Hierdurch ist die Belastung der Herzklappenprothese beim Resheathing geringer. Alle Ausgestaltungen der vorliegenden Erfindung reduzieren jedoch die Kraft, welche beim Wiedereinführen (Resheating) der Herzklappenprothese radial auf die Kapsel wirkt, wodurch das Wiedereinführen der Herzklappenprothese vereinfacht und sicherer gestaltet wird.

Weitere Merkmale, Vorteile und Ausführungsformen der Erfindung sollen nachfolgend anhand der Figuren detailliert erläutert werden. Es zeigen:

Fig. 1    beim resheathing im Stand der Technik auftretende, unvorteilhafte Regenschirmform des Stentgerüstes bzw. der Herzklappenprothese;

Fig. 2    schematische Ansichten einer erfindungsgemäßen Kathetereinrichtung;

Fig. 3    Ansichten einer erfindungsgemäßen Ringstruktur in Form eines einstückigen, selbst-expandierbaren Ringes;

Fig. 4    Ansichten einer erfindungsgemäßen Ringstruktur, die mehrere Ringelemente aufweist;

Fig. 5    eine perspektivische Ansicht einer erfindungsgemäßen Ringstruktur aus mehreren Ringelementen die miteinander verbunden sind;

Fig. 6    Ansichten einer erfindungsgemäßen Ringstruktur aus mehreren Ringelementen die ineinander eingreifen;

Fig. 7    perspektivische Ansichten eines Gelenks einer erfindungsgemäßen Kathetereinrichtung;

Fig. 8    eine perspektivische, ausschnitthafte Ansicht einer erfindungsgemäßen Kathetereinrichtung mit separaten Ringelementen einer Ringstruktur;

Fig. 9    eine perspektivische Ansicht einer äußeren Hülle zum Halten eines Ringelementes bzw. der Stäbe eines Ringelementes einer erfindungsgemäßen Ringstruktur;

Fig. 10   eine perspektivische Ansicht einer Einrichtung zum Bewegen von axial versetzt angeordneten Ringelementen einer erfindungsgemäßen Ringstruktur;

Fig. 11   zeigt verschiedene Ansichten einer Ausführungsform der Erfindung, bei der die Ringstruktur ein Komprimieren der teilweise expandierten Herzklappenprothese/Stentgerüst erlaubt;

Fig. 12   zeigt verschiedene Ansichten einer Modifikation der in der Fig. 11 gezeigten Ausführungsform;

Fig. 13   zeigt in den Schritten a bis d ein Wiedereinziehen (Resheathing) einer teilweise expandierten bzw. freigesetzten Herzklappenprothese, wobei insbesondere der bereits expandierte Abschnitte der Herzklappenprothese bzw. des Stentgerüstes mittels der Ringstruktur (z.B. nach Art der Figuren 11, 12, 15, 16) aktiv komprimiert wird, um das Wiedereinführen in die Kapsel 4 zu erleichtern;

Fig. 14   zeigt verschiedene Ansichten einer Ausführungsform der Erfindung, bei der die Kapsel selber eine im Durchmesser verringerbare Ringstruktur bildet;

Fig. 15   zeigt eine weitere Ausführungsform der Erfindung, bei der die Ringstruktur Stäbe sowie schraubenlinienförmig gekrümmte insbesondere und zwangsgeführte Verbindungsstäbe aufweist; und

Fig. 16 zeigte eine schematische Darstellung der Funktionsweise der Ausführungsform gemäß Fig. 15.

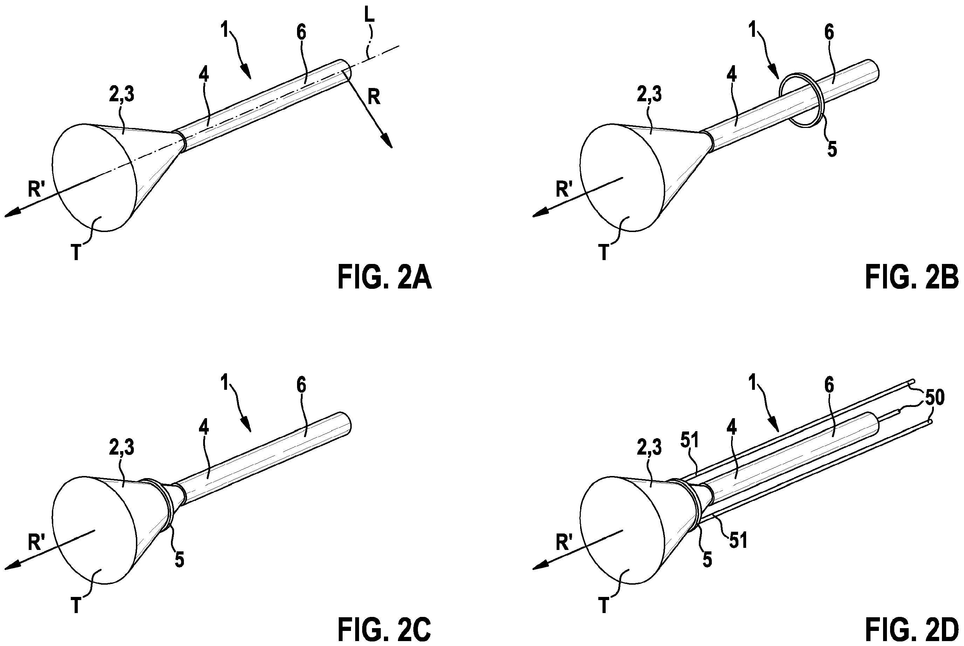

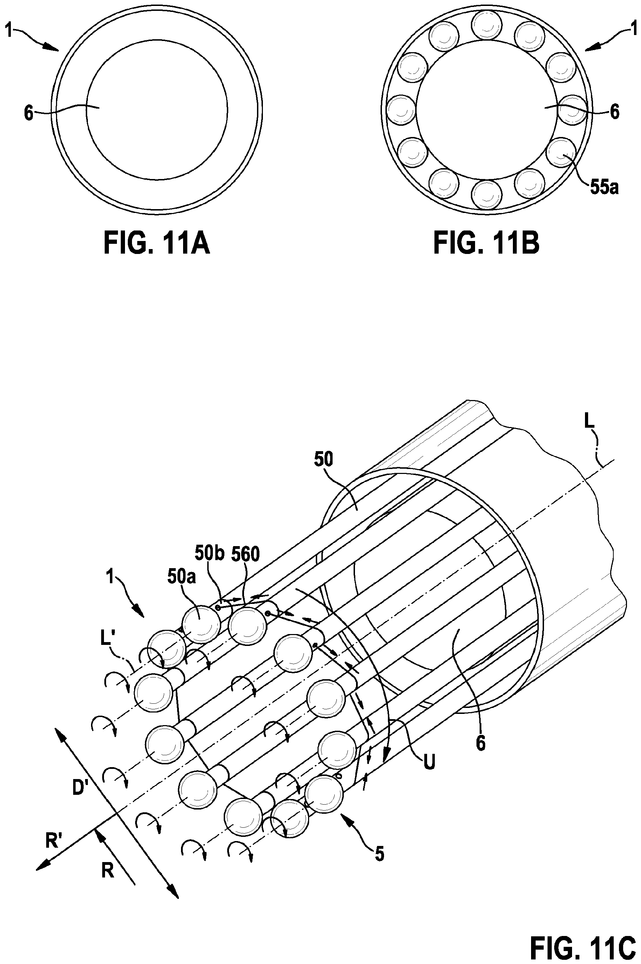

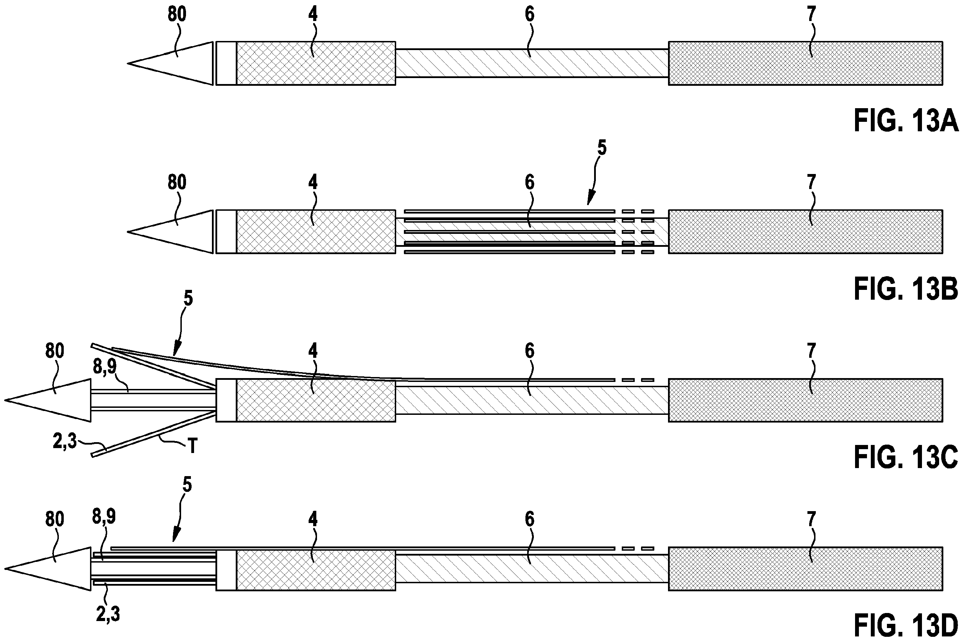

Die Figur 2 zeigt schematische Ansichten einer erfindungsgemäßen Kathetereinrichtung 1 zum Implantieren einer Herzklappenprothese 2. Die Kathetereinrichtung weist hierzu eine Herzklappenprothese 2 auf, die eine Herzklappe aufweist und ein selbst-expandierbares Stentgerüst 3, das die Herzklappe trägt. Zum Transportieren der Herzklappenprothese weist der Katheter 1 weiterhin einen Außenschaft 6 auf (vgl. z.B. auch Fig. 13), der am distalen Ende mit einer Kapsel 4 verbunden ist, die die Herzklappenprothese 2 umgibt. Das distale Ende der Kathetereinrichtung 1 (siehe auch Fig. 13) bildet die Katheterspitze 80 als distales Ende des Innenschaftes 8, wobei die Kapsel 4 und die Herzklappenprothese 2 / Innenschaft 8 relativ zueinander verschiebbar sind, so dass die Herzklappenprothese abschnittsweise freisetzbar ist sowie in die Kapsel 4 (insbesondere vollständig) wieder einführbar ist. Sobald ein Abschnitt T der Herzklappenprothese 2 bzw. des Stentgerüstes 2 freigesetzt wird, entfaltet sich dieser, wie in den Figuren 2a bis 2d und Figur 13 angedeutet ist. Um diesen bereits entfalteten Abschnitt T der Herzklappenprothese 2 ggf. wieder vollständig in die Kapsel 4 einziehen zu können (sogenanntes Resheathing), ohne dass es zu einer übermäßigen regenschirmartigen Verformung (vgl. Figur 1) der Herzklappenprothese 2 bzw. des Stentgerüstes 3 kommt, ist erfindungsgemäß vorgesehen, dass die Kathetereinrichtung 1 eine zusätzliche Ringstruktur 5 aufweist, die in distaler Richtung R' über die Kapsel 4 hinaus verschiebbar ist, so dass der bereits freigesetzte, expandierte Abschnitt T der Herzklappenprothese (insbesondere des Stentgerüstes) mittels der Ringstruktur 5 kontaktierbar ist und insbesondere beim Wiedereinführen in die Kapsel 4 führbar ist (gemäß den weiter unten beschriebenen Ausführungsformen (Figuren 11 bis 16) ist die Ringstruktur 5 auch zum radialen Komprimieren der Herzklappenprothese 2 bzw. des Stentgerüstes 3 ausgelegt).

Somit erlaubt die Erfindung eine Wiedereinführung der Herzklappenprothese 2 in die Kapsel 4 sowie eine erneute Freisetzung der Herzklappenprothese 2 (z.B. wenn die Herzklappenprothese 2 zuvor falsch positioniert war), ohne dass es zu einer übermäßigen Belastung der Herzklappenprothese 2 und der Kapsel 4 bei der Wiedereinführung in die Kapsel 4 kommt.

Die Kathetereinrichtung 1 kann weiterhin einen Innenschaft 8 aufweisen, der in einem Lumen des Außenschaftes 6 angeordnet ist, wobei an einem distalen Ende bzw. der Spitze 80 des Innenschaftes 8 ein Träger 9 vorgesehen ist (vgl. auch Fig. 13), der die Herzklappenprothese 2 trägt. Der Außen- und der Innenschaft 6, 8 sind insbesondere relativ zueinander verschiebbar, so dass der Träger 9 zum Freisetzen bzw. zum abschnittsweise Freisetzen der Herzklappenprothese 2 aus der Kapsel 4 herausführbar ist. Der Außenschaft 6 kann des Weiteren in einem Lumen eines Stabilisierungsschaftes 7 geführt sein, der zur Stabilisierung des Innen- und Außenschaftes 8, 6 dient. Weiterhin kann am Träger 9 eine Befestigungsstruktur vorgesehen sein, die mit einem Teil des Stentgerüstes 3 in Eingriff steht, der z.B. an einem Abschnitt des Stentgerüstes 3 vorgesehen ist, der als letztes freigesetzt wird, so dass eine lediglich teilweise bzw. abschnittsweise freigesetzte Herzklappenprothese 2 wieder in die Kapsel 4 einführbar ist (durch eine entsprechende Relativbewegung des Innenschaftes 8/Trägers 9 und der Kapsel 4). Weiterhin kann der Innenschaft 8 ein Lumen aufweisen in dem ein Führungsdraht verläuft so dass der Innenschaft 8 (mit Träger 9), der Außenschaft 6 (mit Kapsel 4) und der Stabilisierungsschaft 7 durch den Führungsdraht führbar sind.

Wie in der Figur 2d zu erkennen ist, weist die Kathetereinrichtung 1 zum Halten bzw. bewegen der Ringstruktur 5 eine Mehrzahl an Stäben bzw. längs erstreckten Strukturen 50 auf, die sich entlang des Außenschaft des 6 erstrecken und jeweils über einen ersten Endabschnitt 51 mit der Ringstruktur 5 verbunden sind.

Die Stäbe sind dabei dazu konfiguriert die Ringstruktur 5 über die Kapsel 4 hinaus in distaler bzw. axialer Richtung L zu verschieben, so dass die Ringstruktur 5 die teilweise entfaltete Herzklappenprothese 2 (insbesondere das Stentgerüst 3) in der oben beschriebenen Weise kontaktieren und beim Wiedereinführen in die Kapsel 4 führen kann, wobei aufgrund der Ringstruktur eine übermäßige Regenschirmform des Stentgerüstes 3 an der Kapselöffnung verhindert wird.

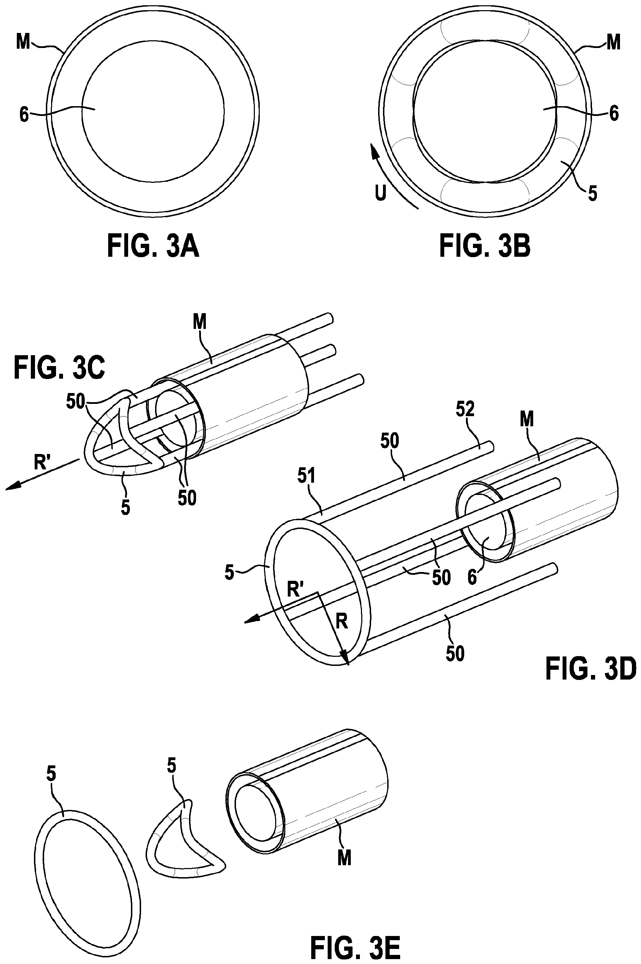

Gemäß den Figuren 3a bis 3e kann die Ringstruktur 5 als ein in sich geschlossener, einstückiger Ring 5 aus einem selbst-expandierbaren Material gebildet sein. In den Figuren 3a bis 3e zeigt die als M bezeichnete Fläche den maximalen Durchmesser (hier exemplarisch 18 F) an, der nicht überschritten werden soll. Der Ring 5 kann gemäß Figuren 2c und 2e auf diesen Durchmesser komprimiert werden, indem die vier Stäbe 50, die den Ring 5 halten, alternierend axial nach vorne (in distaler Richtung R) bzw. nach hinten versetzt angeordnet sind. Nach einem Freisetzen des Ringes 5 entfaltet sich dieser gemäß den Figuren 2d und 2e in seine Kreisform. Gemäß Figur 2d ist zu beachten, dass die Stäbe 50 mit ihren zweiten Endabschnitte in radialer Richtung R nahe des Außenschaftes 6 festgelegt sind, so dass die Stäbe 50 einen gekrümmten Verlauf (insbesondere sförmig gewunden aufweisen).

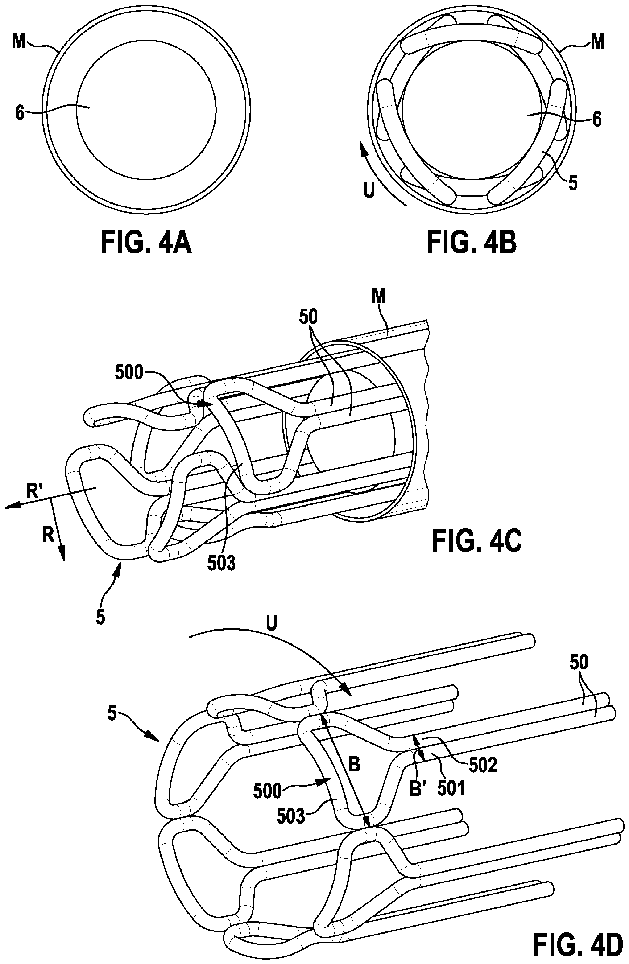

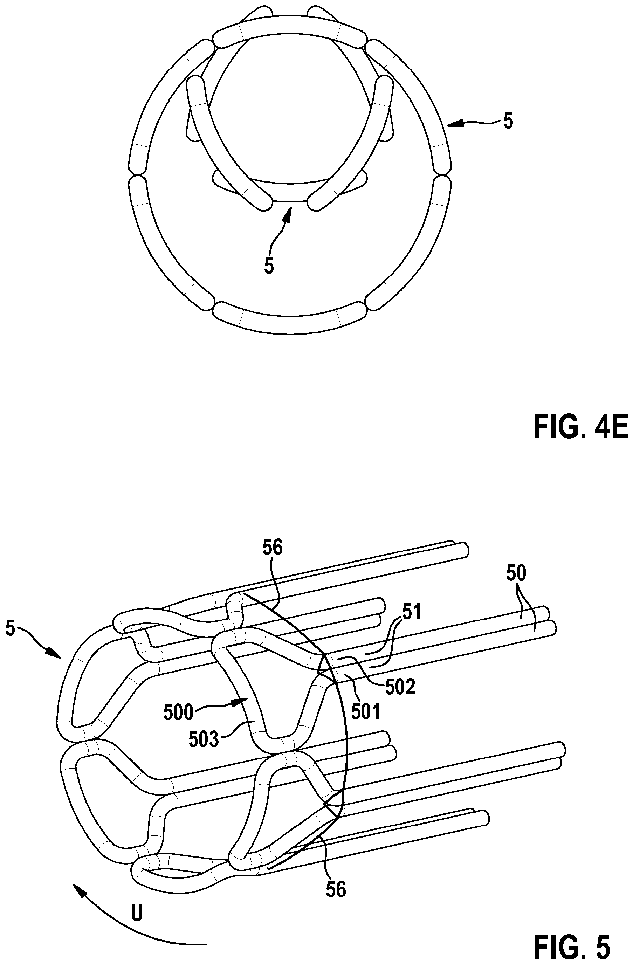

Gemäß einer alternativen Ausführungsform kann die Ringstruktur 5 gemäß den Figuren 4 und 5 auch durch eine Mehrzahl an Ringelementen 500 gebildet sein, wobei die Ringelemente 500 zueinander bewegbar sind, derart, dass die Ringstruktur 5 aus einer ersten Konfiguration heraus (vgl. Fig. 4c und Fig. 4e) in eine zweite Konfiguration (vgl. Fig. 4d und Fig. 4e) bewegbar ist, wobei die Ringstruktur 5 in der zweiten Konfiguration (bezüglich der radialen Richtung R) einen größeren Durchmesser aufweist als in der ersten Konfiguration.

Die Ringelemente 500 sind insbesondere gemäß Fig. 4d nach Art einer geschlossenen Bucht ausgebildet. D.h., die Ringelemente 500 weisen jeweils einen gekrümmten Abschnitt 503 auf, der sich von einem Ende 501 des Ringelementes zu einem anderen Ende 502 des Ringelementes 500 erstreckt, wobei die beiden Enden 501, 502 in Umfangsrichtung U einander gegenüberliegen bzw. parallel ausgerichtet sind und einander kontaktieren. Die Ringelemente 500 weisen hierdurch abseits der Enden eine maximale Breite B in Umfangsrichtung U auf, die größer ist als die Breite B' des Ringelementes 500 an den Enden 501, 502. Hierdurch kann bei entsprechend versetzter Anordnung von benachbarten Ringelementen 500 der Durchmesser der Ringstruktur 5 insgesamt verringert werden, wie in der Fig. 4c gezeigt ist, die die Ringelemente 500 bzw. Ringstruktur 5 in der ersten Konfiguration zeigt. Demgegenüber zeigt die Figur 4d die Ringstruktur 5 in der zweiten Konfiguration, in der die Ringelemente 500 axial auf gleicher Position sowie in Umfangsrichtung U nebeneinander angeordnet sind, was den nunmehr größeren Durchmesser der Ringstruktur 5 bedingt.

Die Stäbe 50 schließen sich jeweils einstückig an die Enden 501, 502 des jeweiligen Ringelementes 500 an, so dass mit jedem Ringelement 500 zwei parallele Stäbe 50 verbunden sind. Da das jeweilige Ringelement 500 integral mit den Stäben 50 verbunden ist, weist die resultierende Struktur 50, 500 keine scharfen Kanten auf, die der Herzklappenprothese 2 gefährlich werden könnten. Um den Durchmesser der Ringstruktur 5 zu begrenzen, können die einzelnen Ringelemente 500 jeweils an den in Umfangsrichtung U benachbarten Ringelementen 500 festgelegt sein, z.B. durch Verknoten der Ringelemente 500 mit einem längserstreckten flexiblen Element 56, das jeweils im Bereich der Enden 501, 502, also am Übergang zu den Stäben 50, festgelegt sein kann, wie es in der Fig. 5 gezeigt ist. Bei dem längserstreckten Element 56 kann es sich z.B. um ein biegeschlaffes Element handeln. Die Stäbe 50 können sich jeweils entlang des Außenschaftes 6 zum Stabilisierungsschaft 7 erstrecken und können an diesem festgelegt sein, so dass die Ringstruktur 5 mittels des Stabilisierungsschafts 7 bewegbar ist, insbesondere vor der Kapsel 4 positionierbar ist.

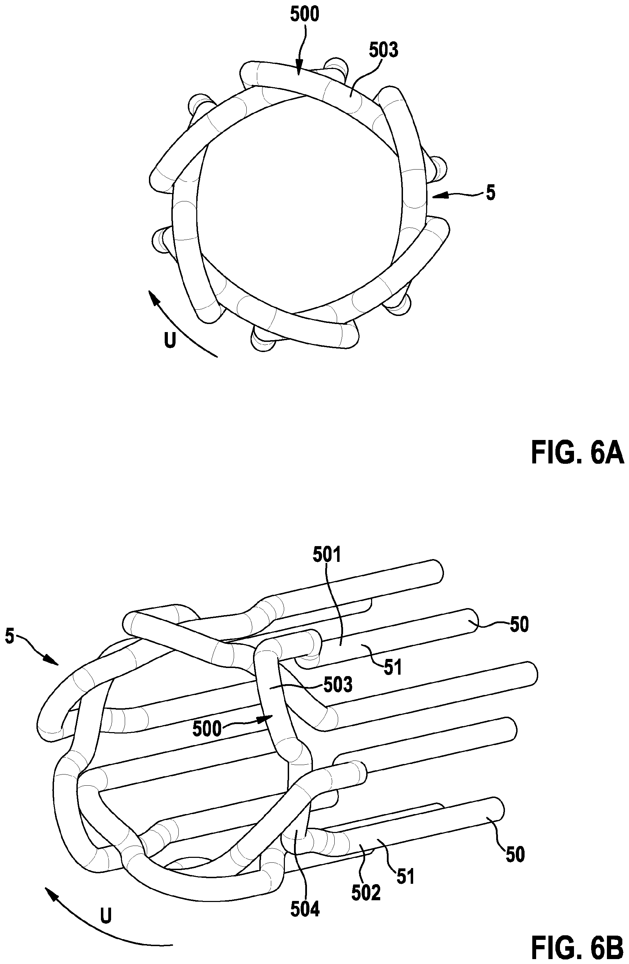

Die Figuren 6a und 6b zeigen eine alternative Ausführungsform der Ringelemente 500, bei der je zwei benachbarte Ringelemente 500 ineinandergreifen, so dass die Ringstruktur 5 durch eine Kette der Ringelemente 500 gebildet ist, wobei die Ringelemente 500 in Umfangsrichtung des Außenschaftes 6 bzw. der Ringstruktur 5 zusammenschiebbar angeordnet sind, so dass der Durchmesser der Ringstruktur entsprechen einstellbar ist. Die Ringelemente 500 sind hier jeweils nach Art einer offenen Bucht ausgebildet. D.h., das jeweilige Ringelement 500 weist einen z.B. im Wesentlichen U-förmig gekrümmten Abschnitt 503 auf, der sich von einem Ende 501 des Ringelementes 500 zu einem in Umfangsrichtung U gegenüberliegenden Ende 502 erstreckt, wobei hier die jeweiligen beiden Enden 501, 502 beabstandet zueinander angeordnet sind. Damit an der der abzustützenden Herzklappenprothese 2 zugewandten Vorderseite der Ringelemente 500 im Wesentlichen eine bündige Oberfläche vorliegt, weisen die Vorderseiten der Abschnitte 503 jeweils eine Stufe 504 auf (vgl. Fig. 6b) über die ein benachbartes Ringelement 500 verläuft.

Auch bei der Ausführungsform gemäß den Figuren 6a, 6b schließt sich an das jeweilige Ende 501, 502 wiederum je ein Stab 50 einstückig an, der sich entlang des Außenschaftes 6 insbesondere zum Stabilisierungsschaft 7 erstreckt und an diesem festgelegt ist, so dass die Ringstruktur 5 mittels des Stabilisierungsschafts 7 bewegbar ist bzw. vor der Kapsel 4 positionierbar ist.

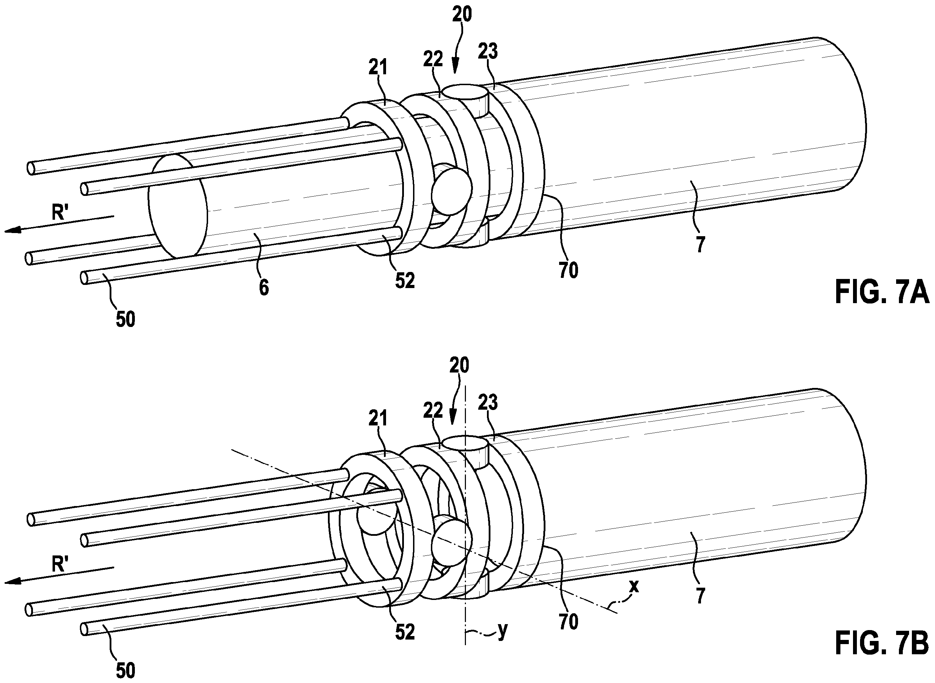

Gemäß der in der Figur 7 gezeigten Ausführungsform einer erfindungsgemäßen Kathetereinrichtung 1 können die Stäbe 50 mit ihren zweiten Endabschnitten 52, die den Ringelementen in der axialen Richtung gegenüberliegen über ein Gelenk 20 mit dem distalen Ende 70 des Stabilisierungsschaftes 7 verbunden sein. Das Gelenk 20 dient zur Kompensation von Verformungen wenn die Schäfte des Katheters (z.B. im Aortenbogen) stark gekrümmt werden. Hierzu ermöglicht das Gelenk 20 eine um zwei orthogonale Achsen x, y kippbare Verbindung der Stäbe 50 mit dem distalen Ende des Stabilisierungsschaftes 7.

Gemäß der in der Figur 7 gezeigten Ausführungsform weist das Gelenk 20 einen ersten Gelenkring 21, einen zweiten Gelenkring 22 sowie einen dritten Gelenkring 23 auf, wobei der zweite Gelenkring 22 zwischen dem ersten und dem dritten Gelenkring 21, 23 angeordnet ist. Dabei umgreifen die drei Gelenkringe 21, 21, 23 jeweils den Außenschaft 6. Zur Herstellung der um die beiden orthogonalen Achsen x, y schwenkbaren Verbindung ist nun vorgesehen, dass der erste Gelenkring 21 um eine erste Achse x kippbar mit dem zweiten Gelenkring 22 verbunden ist, der wiederum um eine zweite orthogonale Achse y kippbar mit dem dritten Gelenkring 23 verbunden ist. Da der erste Gelenkring 21 mit den zweiten Endabschnitten 52 der Stäbe 50 und der dritte Gelenkring 23 mit dem distalen Ende 70 des Stabilisierungsschaftes 7 starr verbunden ist, ergibt sich eine entsprechend gelenkige Festlegung der Stäbe 50 am distalen Ende des Stabilisierungsschafts 7.

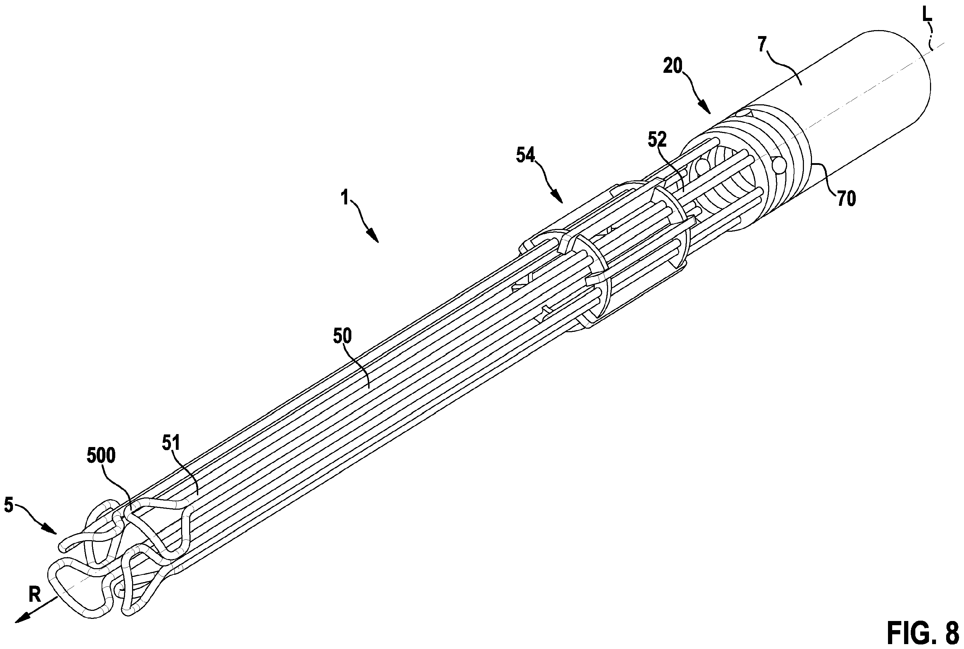

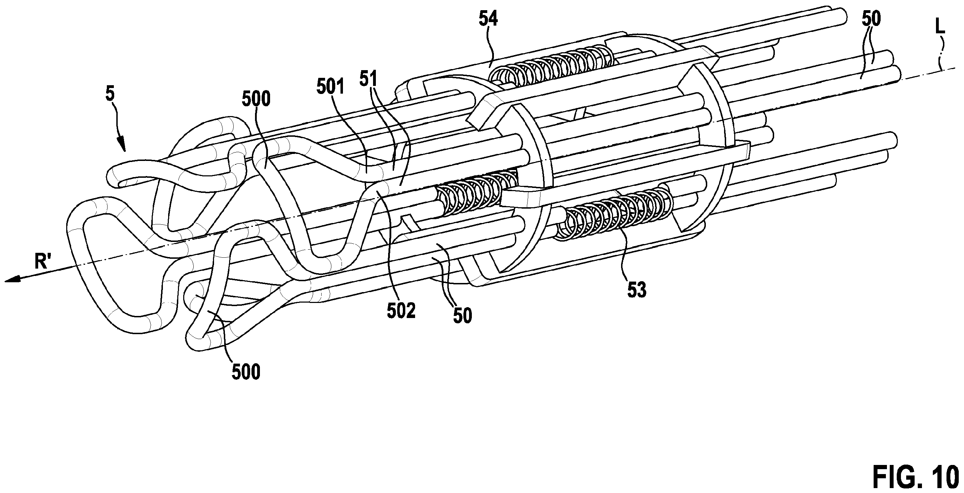

Bei Ringelementen 500, die in axialer L bzw. distaler Richtung R' initial alternierend versetzt angeordnet sind (siehe oben), ist des Weiteren gemäß den Figuren 8 und 10 vorgesehen, dass diejenigen Ringelemente 500, die in distaler Richtung R' weiter vorne angeordnet sind (vgl. auch Fig. 4c) mit Stäben 50 verbunden sind, die einen in axialer Richtung L elastisch komprimierbaren Abschnitt 53 aufweisen, der vorliegend insbesondere durch je eine Feder 53 gebildet ist. Zum Führen dieser Stäbe 50 ist des Weiteren eine Führung 54 vorgesehen, die den Außenschaft 7 umgreift, wobei jeweils der zwischen der Feder 53 und dem entsprechenden Ringelement 500 erstreckte Abschnitt eines solchen Stabes 50 so durch die Führung 54 geführt wird, dass er bezüglich der Führung in axialer Richtung L gleiten kann. Die nicht federbaufschlagten Ringelemente 500 bzw. Stäbe 50 sind hingegen insbesondere starr mit der Führung 54 gekoppelt.

Wird nun die Ringstruktur 5 in distaler Richtung R' über die Kapsel 4 hinaus verschoben, werden die weiter vorne gelegenen Ringelemente 500 bei einem Kontakt mit der abschnittsweise freigesetzten Herzklappenprothese 2 gegen eine rückstellende Kraft des jeweiligen elastisch komprimierbaren Abschnittes (z.B. Feder) 53 zurückgeschoben, bis alle Ringelemente 500 die Herzklappenprothese 2 kontaktieren. Hierbei erfolgt automatisch auch eine Aufweitung der Ringstruktur 5 auf den größeren Durchmesser (zweite Konfiguration, siehe auch oben).

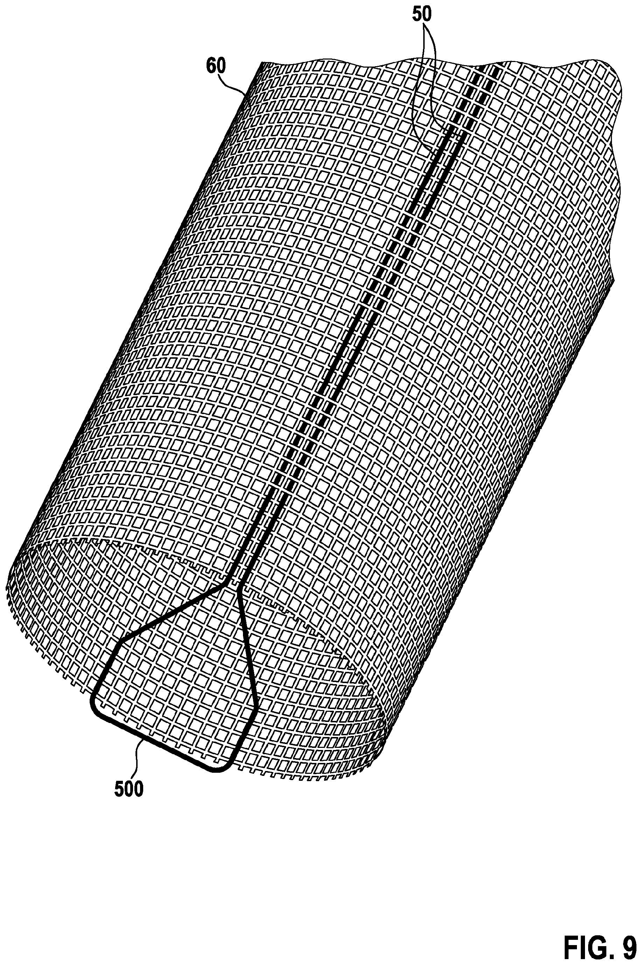

Schließlich können die Stäbe 50 von einer äußeren, den Außenschaft 6 umgebenden Hülle 60 umgeben sein, wobei die Stäbe 50 auch gemäß Fig. 9 in eine solche Hülle 60 eingefädelt sein können. Die Stäbe 50 durchstoßen hierbei die Hülle 60 an mehreren Stellen, so dass sie alternierend unterhalb und oberhalb der Hülle 60 verlaufen. Mittels der Hülle 60 kann somit der Durchmesser der Ringstruktur auf einen maximalen Durchmesser begrenzt werden. Eine solche Hülle kann z.B. aus einem Dacron®-Gewebe oder einem anderen geeigneten Material bestehen.

Die Figuren 11 bis 16 zeigen des Weiteren Ausführungsformen der vorliegenden Erfindung, die ein aktives Komprimieren der bereits teilweise expandierten bzw. freigesetzten Herzklappenprothese 2 bzw. des Stentgerüstes 3 erlauben,

Das in der Figur 13 (a bis d) gezeigte Resheathing oder Wiedereinziehen eines teilweise expandierten Abschnitts T einer Herzklappenprothese 2 erfordert insbesondere, dass der Durchmesser des Stentgerüstes 3 deutlich reduzierbar ist (üblicherweise um einen Faktor 3 bis 4), so dass die Herzklappenprothese 2 wieder in der Kapsel 4 aufnehmbar ist. Im Stand der Technik wird dies üblicherweise durch Einleiten einer axialen Kraft (Ziehen) erreicht, was die Herzklappenprothese 2 und die Kapsel 4 stark belastet und hohe Reibungskräfte erzeugt.

Gemäß Figur 11 (a bis c) ist vorgesehen, dass die Kathetereinrichtung 1 eine Ringstruktur 5 aufweist, die in distaler R' bzw. axialer Richtung L verschiebbar ist, und zwar insbesondere über einen bereits freigesetzten, expandierten Abschnitt T der Herzklappenprothese 2, so dass dieser mittels der Ringstruktur 5 komprimierbar ist.

Die Ringstruktur 5 weist hierzu gemäß Figur 11 (b und c) einen verringerbaren Durchmesser D' auf, so dass die Ringstruktur 5 um die teilweise entfaltete Herzklappenprothese 2 zusammenziehbar ist und dabei eine Kraft in radialer Richtung R auf die Herzklappenprothese 2 ausüben kann, die die Herzklappenprothese 2, insbesondere das Stentgerüst 3, in radialer Richtung R komprimiert, so dass die Herzklappenprothese 2 wieder in die Kapsel 4 zurückbewegbar ist (Resheathing).

Insbesondere ist gemäß Fig. 11 (b und c) vorgesehen, dass die Ringstruktur 5 eine Mehrzahl an Stäben 50 aufweist, die sich in distaler R' bzw. axialer Richtung L erstrecken, sowie ein längserstrecktes flexibles Element 560. Die Stäbe 50 weisen jeweils ein erstes Ende 50a auf, wobei die Stäbe 50 am ersten Ende 50a jeweils eine Durchgangsöffnung 50b aufweisen, und wobei das längserstreckte flexible Element 560 durch die Durchgangsöffnungen 50b geführt ist.

Bei dem Element 560 kann es sich um ein biegeschlaffes Element handeln. Weiterhin kann es sich bei dem Element 560 um einen Draht, einen Faden, oder ein Seil handeln. Das Element 560 bildet insbesondere einen in sich geschlossenen Ring aus, so dass ein (synchrones und gleichgerichtetes) Rotieren der Stäbe 50 um die Längsachse L' der Stäbe 50 dazu führt, dass sich das flexible Element 560 um den jeweiligen Stab 50 wickelt, so dass die Ringstruktur 5 im Durchmesser D' verringert wird, sich dabei um die teilweise expandierte Herzklappenprothese 2 zusammenzieht und diese in radialer Richtung R zum Wiedereinführen in die Kapsel 4 komprimiert.

Die Verringerung des Durchmessers D' beim besagten Rotieren der Stäbe 50 wird insbesondere dadurch erzeugt, dass der Abstand benachbarter Stäbe 50 in Umfangsrichtung U der Ringstruktur 5 verringert wird. Der erfindungsgemäße Mechanismus wandelt also hier ein Drehmoment/Torsion in eine die Herzklappenprothese 2 radial komprimierende Bewegung der Ringstruktur 5 um. In dieser Weise können hohe radiale Kräfte mit vergleichsweise niedrigen Drehmomenten erzielt werden, da die Hebelarme verhältnismäßig kurz sind:

$$F=M/r$$

F: Kraft in Umfangsrichtung zwischen benachbarten Stäben 50, die in radiale Kraft übersetzt wird.
M: Auf den jeweiligen Stab 50 wirkendes Drehmoment.
r: Radius des jeweiligen Stabes 50 (Hebelarm).

Für den Fall, dass das Element 560 lose ist, können die insbesondere flexiblen Stäbe 50 sich in der radialen Richtung verformen/verbiegen (in Bezug auf die Mittellinie des Katheters bzw. die Längsachse L), wodurch die Stäbe 50 leicht in distaler Richtung R' über die Kapsel 4 und die teilweise expandierte Herzklappenprothese 2 bewegbar sind (siehe auch Fig. 13 c).

Die Stäbe 50 können insbesondere mittels des Stabilisierungsschaftes 7 in distaler Richtung R' verschiebbar sein. Die Stäbe 50 können weiterhin über ihre zweiten Enden, die den ersten Enden 50a gegenüberliegen, mit einem Mechanismus gekoppelt sein, mittels dem die Stäbe 50 um die jeweilige Längsachse L' (synchron und jeweils gleichgerichtet) rotierbar sind.

Wie in der Figur 11 dargestellt, sind die ersten Enden 50a bevorzugt kugelförmig ausgebildet, wodurch mögliche Beschädigungen der Herzklappenprothese 2 durch die ersten Enden 50a verhindert werden.

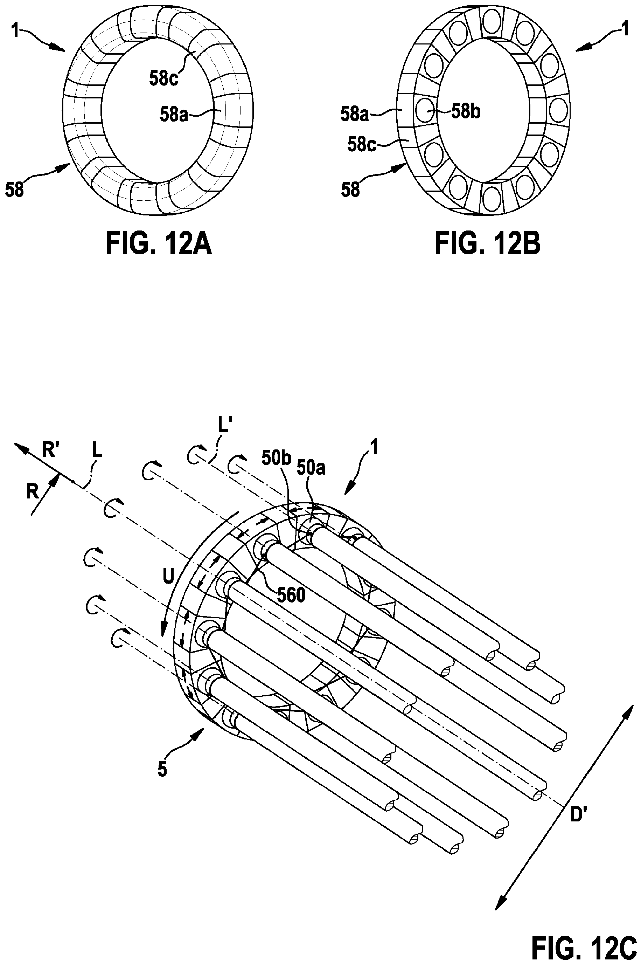

Gemäß der Figur 12 können die ersten Enden 50a in einem ringförmigen Element 58 der Ringstruktur 5 gelagert sein, wobei die ersten Enden 50a der Stäbe 50 jeweils in eine zugeordnete Ausnehmung 58b des ringförmigen Elementes 58 eingreifen und dort um die Längsachse L' des jeweiligen Stabes 50 rotierbar gelagert sind. Die jeweilige Ausnehmung 58b bildet dabei ein Drehlager bzw. Gleitlager für das erste Ende 50a des jeweiligen Stabes 50.

Die Ausnehmungen bzw. Gleitlager 58b können gemäß Fig. 12 (b und c) jeweils in einem starren Abschnitt 58a des ringförmigen Elementes 58 angeordnet sein, wobei je zwei in Umfangsrichtung U benachbarte starre Abschnitte 58a durch einen Zwischenabschnitt 58c miteinander verbunden sind, der z.B. elastisch deformierbar ausgebildet sein kann. Durch Rotieren der Stäbe 50 können die elastisch deformierbaren Zwischenabschnitte 58c in Umfangsrichtung U des ringförmigen Elementes 58 zusammengedrückt werden, wobei der Durchmesser D' des ringförmigen Elementes 58 verringert wird. Auch hier werden nämlich die Stäbe 50 aufgrund des ringförmig durch die Durchgangsöffnungen 50b geführten flexiblen Elementes 560 in Umfangsrichtung U aneinander angenähert, wenn die Stäbe 50 synchron um ihre jeweilige Längsachse L' (in die gleiche Richtung) rotiert werden.

Die Zwischenabschnitte 58c erlauben des Weiteren eine Vergrößerung des Durchmessers D' der Ringstruktur 5 bzw. des ringförmigen Elementes 58, nämlich wenn die Ringstruktur 5 bei losem Element 560 in distaler Richtung R' über die Kapsel 4 hinweg geschoben wird, um die teilweise expandierte Herzklappe 2 zum Komprimieren der Herzklappe 2 zu umgreifen. Ein anschließendes Rotieren der Stäbe 50 (siehe oben) führt dann dazu, dass sich die Ringstruktur 5 bzw. das ringförmige Element 58 um die teilweise expandierte Herzklappenprothese 2 (bzw. das Stentgerüst 3) zusammenzieht und diese in radialer Richtung zum Wiedereinziehen in die Kapsel 4 komprimiert.

Die Zwischenabschnitte 58c stellen insbesondere eine mechanische Kopplung/Verbindung der starren Abschnitte 58a bzw. Lager 58b her, so dass die starren Abschnitte 58a / Lager 58b relativ zueinander in der Umfangsrichtung U der Ringstruktur 5 bewegbar sind. Abgesehen von den oben beschriebenen elastisch deformierbaren Zwischenelementen 58c, können die Zwischenelemente 58c insbesondere als Federelemente ausgebildet sein, oder als Elemente die zumindest oder lediglich eine gleitende Bewegung der starren Abschnitte 58a / Lager 58b zueinander in Umfangsrichtung U gestatten. Die Zwischenabschnitte 58c können insbesondere aus einem anisotropen Material gefertigt sein, das insbesondere nur in der Umfangsrichtung U flexibel ist, nicht jedoch in den anderen Richtungen (z.B. quer zur Umfangsrichtung U).

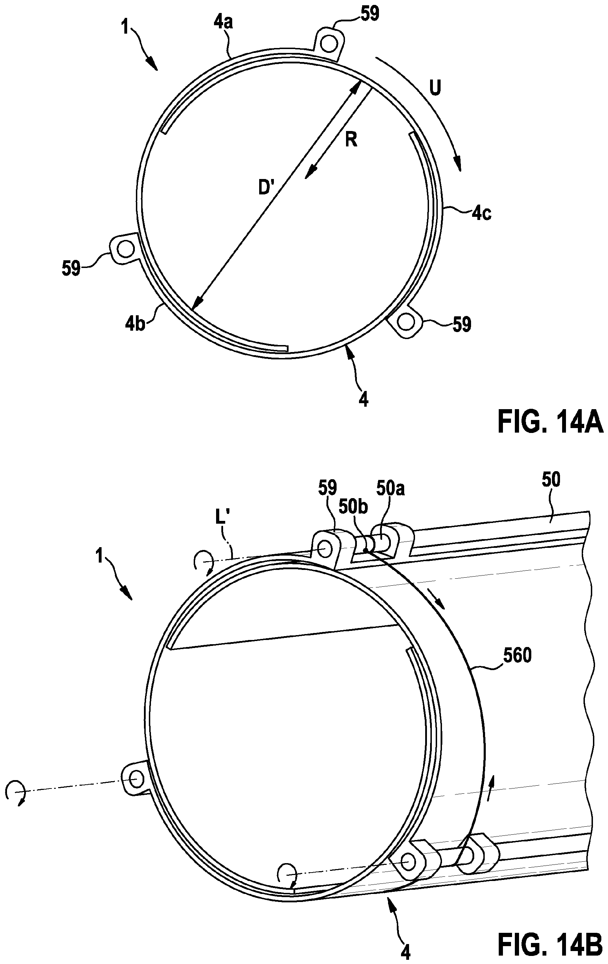

Weiterhin zeigt Figur 14 eine Ausführungsform, bei der die Kapsel 4 selbst eine aktiv im Durchmesser D' veränderliche Ringstruktur bildet. Hier wird also im Unterschied zu den anderen Ausführungsformen keine zusätzliche Ringstruktur 5 über die Kapsel 4 hinweg über die teilweise expandierte Herzklappenprothese 2 geschoben, sondern ein entsprechender Mechanismus ist in die Kapsel 4 integriert.

Die Kapsel 4 weist in diesem Beispiel drei (z.B. konvex gekrümmte bzw. schalenförmige) Wandelemente 4a, 4b, 4c auf, wobei in Umfangsrichtung U der Kapsel 4 benachbarte Wandelemente 4a, 4b, 4c einander überlappen, so dass eine umlaufende Wandstruktur vorliegt. Weiterhin weist jedes Wandelement 4a, 4b, 4c ein Lager 59 für einen ersten Endabschnitt 50a eines Stabes 50 auf, der sich in distaler R' bzw. axialer Richtung L erstreckt, wobei jeder Stab 50 in seinem Lager 59 um seine Längsachse L' rotierbar ist. Die Stäbe 50 weisen wiederum jeweils eine Durchgangsöffnung 50b auf, durch die ein längserstrecktes flexibles Element 560 geführt ist.

Bei dem Element 560 kann es sich wiederum um ein biegeschlaffes Element handeln. Weiterhin kann es sich bei dem Element 560 um einen Draht, einen Faden, oder ein Seil handeln. Das Element 560 bildet insbesondere einen in sich geschlossenen Ring, so dass ein (synchrones und gleichgerichtetes) Rotieren der Stäbe 50 um die Längsachse L' der Stäbe 50 dazu führt, dass sich das flexible Element 560 um den jeweiligen Stab 50 wickelt, so dass je zwei einander überlappende Wandelemente 4a, 4b, 4c in Umfangsrichtung U übereinander geschoben werden bzw. stärker überlappen, wobei die die Wandelemente 4a, 4b, 4c aufweisende Kapsel bzw. Ringstruktur 4 im Durchmesser D' verringert wird und sich dabei um die teilweise expandierte Herzklappenprothese zusammenzieht und diese in radialer Richtung R zum Wiedereinführen in die Kapsel 4 komprimiert (vgl. Fig. 14 a). Die Stäbe 50 können wiederum über ihre zweiten Endabschnitte (nicht gezeigt), die den ersten Endabschnitten 50a gegenüberliegen, mit einem Mechanismus gekoppelt sein, mittels dem die Stäbe 50 um die jeweilige Längsachse L' rotierbar sind.

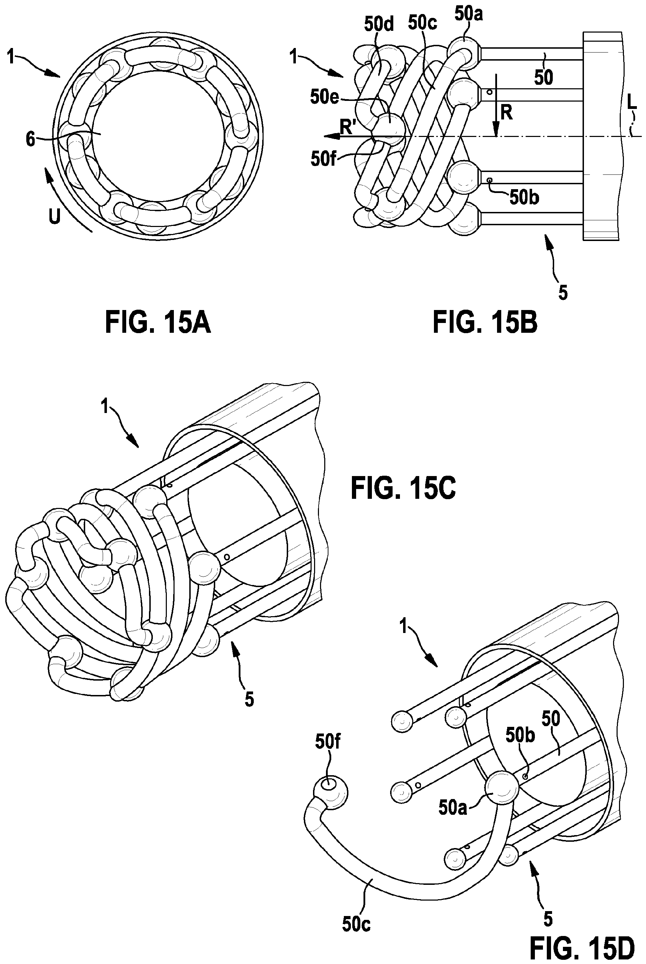

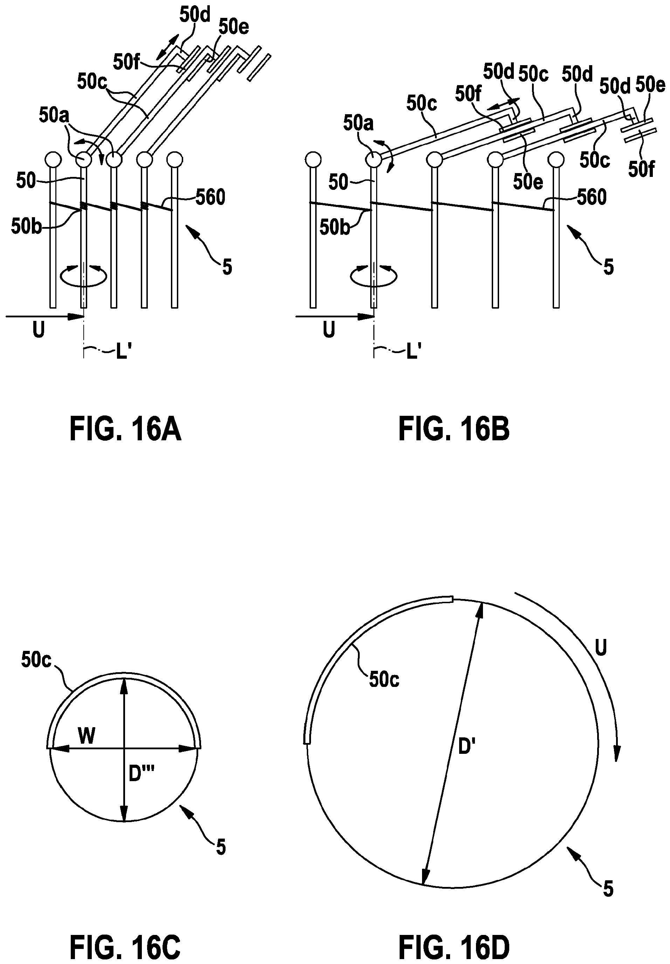

Figur 15 zeigt im Zusammenhang mit der Figur 16 eine weitere Ausführungsform einer im Durchmesser D' verringerbaren Ringstruktur 5 zum radialen Komprimieren einer teilweise expandierten Herzklappenprothese 2.

Hierbei ist vorgesehen, dass die Ringstruktur 5 eine Mehrzahl an Stäben 50 aufweist, die sich in distaler R' bzw. axialer Richtung L erstrecken, sowie ein längserstrecktes flexibles Element 560. Die Stäbe 50 weisen jeweils ein erstes Ende 50a auf, an dem der jeweilige Stab 50 eine Durchgangsöffnung 50b aufweist, wobei das längserstreckte flexible Element 560 durch die Durchgangsöffnungen 50b geführt ist. Die ersten Enden 50a können jeweils kugelförmig ausgebildet sein.

Weiterhin ist jedes erste Ende 50a mit einem schraubenlinienförmig gekrümmten Verbindungsstab 50c verbunden (insbesondere einstückig), wobei der jeweilige Verbindungstab 50c einen abgewinkelten Endabschnitt 50d aufweist, der in einem kugelförmigen Ende 50e endet, das eine als Gleitlager ausgestaltete Durchgangsöffnung 50f aufweist, wobei in der jeweiligen Durchgangsöffnung 50f ein benachbarter Verbindungstab 50c gleitend gelagert ist.

Bei dem längserstreckten flexiblen Element 560 kann es sich wiederum um ein biegeschlaffes Element 560 handeln. Weiterhin kann es sich bei dem Element 560 um einen Draht, einen Faden, oder ein Seil handeln. Das Element 560 bildet insbesondere einen in sich geschlossenen Ring, so dass ein Rotieren der Stäbe 50 um die Längsachse L' der Stäbe 50 dazu führt, dass sich das flexible Element 560 um den jeweiligen Stab 50 wickelt.

Die Stäbe 50 rücken hierbei gemäß der zweidimensionalen Projektion, die in der Figur 16 (a und b) dargestellt ist, näher zusammen (Wechsel von Fig. 14b nach Fig. 14 a), wobei die Gleitlager 50f der Verbindungsstäbe 50c zum abgewinkelten Endabschnitt des jeweils geführten Verbindungsstabes 50c hin verschoben werden. Entsprechend verringert sich der Durchmesser D' der Ringstruktur zu dem geringeren Durchmesser D", wie es in der Figur 16c angedeutet ist.

Die Stäbe 50 bzw. Verbindungstäbe 50c können sich somit um die teilweise expandierte Herzklappenprothese 2 durch (synchrones und gleichgerichtetes) Rotieren der Stäbe 50 um deren Längsachsen L' zusammenziehen, wobei die Herzklappenprothese 2 in radialer Richtung R zum Wiedereinführen in die Kapsel 4 komprimiert wird. Eine entgegengesetzte Rotation erlaubt entsprechend eine Vergrößerung des Durchmessers der Ringstruktur 5 (z.B. um diese in distaler Richtung R' über die Kapsel 4 hinaus auf die teilweise expandierte Herzklappenprothese 2 zu schieben).

Die Stäbe 50 können wiederum über ihre zweiten Enden (nicht gezeigt), die den ersten Enden 50a gegenüberliegen, mit einem Mechanismus gekoppelt sein, mittels dem die Stäbe 50 um die jeweilige Längsachse L' rotierbar sind.

Die kugelförmigen Enden 50a und 50e können durch Kugeln gebildet sein, die z.B. durch eine Pressverbindung oder eine Klebeverbindung mit dem jeweiligen Stab 50 bzw. dem jeweiligen Verbindungsstab 50 c verbunden sind.

Die besagten Kugeln können z.B. aus einem Saphir, einem Rubin oder eine Keramik geformt sein. Die Stäbe und Verbindungsstäbe bestehen bevorzugt aus einem Metall Wie beispielsweise rostfreien Edelstahl. Die Kugeln können z.B. einen Durchmesser von 0,9mm aufweisen. Der Durchmesser der Verbindungsstäbe kann z.B. 0,5 mm betragen. Die schraubenlinienförmigen Verbindungsstäbe können z.B. eine Länge aufweisen, dass ein entsprechender Kreissektorwinkel etwa W=180° beträgt, wenn die Ringstruktur 5 einen minimalen Durchmesser D" aufweist (z.B. gemäß dem Beispiel in Fig. 16 c). Die Länge der Verbindungsstäbe 50c kann variieren und wird so gewählt, dass eine gewünschte Durchmesserverringerung der Ringstruktur 5 erfolgen kann.

**Patentansprüche**

**1.** Kathetereinrichtung (1) zum Implantieren einer Herzklappenprothese (2), mit:

- einer Herzklappenprothese (2), die eine Herzklappe sowie ein selbst-expandierbares Stentgerüst (3) aufweist, das die Herzklappe trägt,
- einem Außenschaft (6), der an einem distalen Ende eine Kapsel (4) aufweist, die die Herzklappenprothese (2) umgibt, wobei die Kapsel (4) und die Herzklappenprothese (2) relativ zueinander verschiebbar sind, so dass die Herzklappenprothese (2) abschnittsweise freisetzbar ist, wobei ein freigesetzter Abschnitt (T) des Stentgerüsts (3) sich selbständig expandiert, sowie in die Kapsel (4) wieder einführbar ist,

**dadurch gekennzeichnet,**
**dass** die Kathetereinrichtung (1) eine Ringstruktur (5) aufweist, die über einen bereits freigesetzten, expandierten Abschnitt der Herzklappenprothese (2) positionierbar ist, so dass der bereits freigesetzte, expandierte Abschnitt der Herzklappenprothese (2) mittels der Ringstruktur (5) kontaktierbar und/oder komprimierbar ist.

**2.** Kathetereinrichtung nach Anspruch 1, **dadurch gekennzeichnet, dass** die Ringstruktur (5) dazu ausgebildet ist, einen Durchmesser des Stentgerüstes (3) am Ort der Ringstruktur (5) zu begrenzen, so dass entsprechend eine axiale Kraft, die durch die Kapsel (4) beim Wiedereinführen der Herzklappenprothese (2) in die Kapsel (4) auf das Stentgerüst (3) ausgeübt wird, begrenzt wird.

**3.** Kathetereinrichtung nach Anspruch 1 oder 2, **dadurch gekennzeichnet, dass** die Kathetereinrichtung (1) zum Tragen der Ringstruktur (5) eine Mehrzahl an Stäben (50) aufweist, die jeweils über einen ersten Endabschnitt (51) mit der Ringstruktur (5) verbunden sind.

**4.** Kathetereinrichtung nach einem der vorhergehenden Ansprüche, **dadurch gekennzeichnet, dass** die Kathetereinrichtung (1) einen äußeren Stabilisierungsschaft (7) zum Stabilisieren des Außenschaftes (6) aufweist, wobei der Außenschaft (6) in einem vom Stabilisierungsschaft (7) umgebenden Lumen des Stabilisierungsschaftes (7) angeordnet ist, und wobei der Außenschaft (6) und der Stabilisierungsschaft (7) relativ zueinander verschiebbar sind.

**5.** Kathetereinrichtung nach Anspruch 4, **dadurch gekennzeichnet, dass** die Stäbe (50) jeweils über einen zweiten Endabschnitt (52) an einem distalen Ende (70) des Stabilisierungsschaftes (7) festgelegt sind.

**6.** Kathetereinrichtung nach Anspruch 5, **dadurch gekennzeichnet, dass** die zweiten Endabschnitte (52) der Stäbe über ein Gelenk (20) mit dem distalen Ende (70) des Stabilisierungsschaftes (7) verbunden sind.

**7.** Kathetereinrichtung nach Anspruch 6, **dadurch gekennzeichnet, dass** das Gelenk (20) einen ersten Gelenkring (21), einen zweiten Gelenkring (22) sowie einen dritten Gelenkring (23) aufweist, wobei der zweite Gelenkring (22) zwischen dem ersten und dem dritten Gelenkring (21, 23) angeordnet ist, und wobei die drei Gelenkringe (21, 21, 23) den Außenschaft (6) umfassen, wobei der erste Gelenkring (21) um eine erste Achse (x) kippbar mit dem zweiten Gelenkring (22) verbunden ist, und wobei der zweite Gelenkring (22) um eine zweite Achse (y) kippbar mit dem dritten Gelenkring (23) verbunden ist, wobei die beiden Achsen (x, y) orthogonal zueinander verlaufen, und wobei der erste Gelenkring (21) mit den zweiten Endabschnitten (52) der Stäbe (50) starr verbunden ist, und wobei der dritte Gelenkring (23) mit dem distalen Ende (70) des Stabilisierungsschaftes (7) verbunden ist.

**8.** Kathetereinrichtung nach einem der vorhergehenden Ansprüche, **dadurch gekennzeichnet, dass** die Ringstruktur (5) ein durchgehender, in sich geschlossener und selbst-expandierender Ring ist.

**9.** Kathetereinrichtung nach einem der Ansprüche 1 bis 7, **dadurch gekennzeichnet, dass** die Ringstruktur (5) durch eine Mehrzahl an Ringelementen (500) gebildet ist, wobei die Ringelemente (500) zueinander bewegbar sind, derart, dass die Ringstruktur (5) aus einer ersten Konfiguration heraus in eine zweite Konfiguration bewegbar ist, wobei die Ringstruktur (5) in der zweiten Konfiguration einen größeren Durchmesser aufweist als in der ersten Konfiguration.

**10.** Kathetereinrichtung nach den Ansprüchen 3 und 9, **dadurch gekennzeichnet, dass** jeder Stab (50) über seinen ersten Endabschnitt (51) mit einem Ringelement (500) verbunden ist.

**11.** Kathetereinrichtung nach Anspruch 9 oder 10, **dadurch gekennzeichnet, dass** je zwei benachbarte Ringelemente (500) ineinandergreifen.

**13.** Kathetereinrichtung nach Anspruch 9 oder 10, **dadurch gekennzeichnet, dass** je zwei benachbarte Ringelemente (500) in der ersten Konfiguration axial zueinander versetzt angeordnet sind, so dass die Ringstruktur (5) erste Ringelemente (500) aufweist, die in der ersten Konfiguration der Ringstruktur (5) in distaler Richtung (R') vor zweiten Ringelementen (500) der Ringstruktur (5) angeordnet sind, und dass in der zweiten Konfiguration die Ringelemente (500) in der Umfangsrichtung (U) nebeneinander angeordnet sind.

**14.** Kathetereinrichtung nach den Ansprüchen 11 und 13, **dadurch gekennzeichnet, dass** die mit den ersten Ringelementen (500) verbundenen Stäbe (50) einen in axialer Richtung elastisch komprimierbaren Abschnitt (53) aufweisen, so dass, wenn die Ringstruktur (5) in distaler Richtung (R') verschoben wird, die ersten Ringelemente (500) bei einem Kontakt mit der abschnittsweise freigesetzten Herzklappenprothese (2) gegen eine rückstellende Kraft des jeweiligen elastisch komprimierbaren Abschnittes (53) zurückgeschoben werden, bis alle Ringelemente (500) die Herzklappenprothese (2) kontaktieren.

**15.** Kathetereinrichtung nach Anspruch 3 oder einem der Ansprüche 4 bis 14 soweit rückbezogen auf Anspruch 3, **dadurch gekennzeichnet, dass** die Stäbe (50) von einer äußeren, den Außenschaft (6) umgebend Hülle (60), Draht oder Bandmaterial umgeben sind oder an einer äußeren, den Außenschaft (6) umgebenden Hülle (60), Draht oder Bandmaterial festgelegt sind.

FIG. 1
(Stand der Technik)

EP 3 501 453 A1

FIG. 2B

FIG. 2D

FIG. 2A

FIG. 2C

**FIG. 3A**

**FIG. 3B**

**FIG. 3C**

**FIG. 3D**

**FIG. 3E**

FIG. 4A

FIG. 4B

FIG. 4C

FIG. 4D

FIG. 4E

FIG. 5

FIG. 6A

FIG. 6B

**FIG. 7A**

**FIG. 7B**

EP 3 501 453 A1

FIG. 8

FIG. 9

FIG. 10

**FIG. 11A**

**FIG. 11B**

**FIG. 11C**

**FIG. 12A**

**FIG. 12B**

**FIG. 12C**

FIG. 13A

FIG. 13B

FIG. 13C

FIG. 13D

**FIG. 14A**

**FIG. 14B**

**FIG. 15A**

**FIG. 15B**

**FIG. 15C**

**FIG. 15D**

FIG. 16A

FIG. 16B

FIG. 16C

FIG. 16D

**Europäisches Patentamt**
**European Patent Office**
**Office européen des brevets**

# EUROPÄISCHER RECHERCHENBERICHT

**Nummer der Anmeldung**

EP 17 20 9494

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | WO 2010/042950 A2 (SADRA MEDICAL INC [US]; PAUL DAVID [US]; SUTTON BENJAMIN [US]; MCCOLLU) 15. April 2010 (2010-04-15) | 1-5, 9-11, 13-15 | INV. A61F2/24 A61F2/95 |
| Y | * Absätze [0055] - [0057], [0107] - [0111]; Abbildungen 2a,24-28 * ----- | 8 | |
| Y | US 2012/123515 A1 (HOSFORD ANDREW T [US] ET AL) 17. Mai 2012 (2012-05-17) | 8 | |
| A | * Absätze [0016] - [0033]; Abbildungen 1-4 * ----- | 3-7 | |
| A | US 2007/239254 A1 (CHIA CHRIS [US] ET AL) 11. Oktober 2007 (2007-10-11) * Absätze [0007], [0008], [0011], [0036], [0037], [0060] - [0063]; Abbildungen 12A-14B * ----- | 1-11, 13-15 | |

**RECHERCHIERTE SACHGEBIETE (IPC)**

A61F

Der vorliegende Recherchenbericht wurde für alle Patentansprüche erstellt

| Recherchenort | Abschlußdatum der Recherche | Prüfer |
|---|---|---|
| Den Haag | 30. Mai 2018 | Geuer, Melanie |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

&: Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 17 20 9494

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

30-05-2018

| Im Recherchenbericht angeführtes Patentdokument | Datum der Veröffentlichung | Mitglied(er) der Patentfamilie | Datum der Veröffentlichung |
|---|---|---|---|
| WO 2010042950 A2 | 15-04-2010 | CA 2739961 A1 | 15-04-2010 |
| | | CN 102245256 A | 16-11-2011 |
| | | EP 2340075 A2 | 06-07-2011 |
| | | EP 2617388 A2 | 24-07-2013 |
| | | EP 3238661 A1 | 01-11-2017 |
| | | ES 2409693 T3 | 27-06-2013 |
| | | ES 2627860 T3 | 31-07-2017 |
| | | JP 5607639 B2 | 15-10-2014 |
| | | JP 2012505061 A | 01-03-2012 |
| | | US 2010121434 A1 | 13-05-2010 |
| | | US 2010280495 A1 | 04-11-2010 |
| | | US 2012046740 A1 | 23-02-2012 |
| | | US 2014114405 A1 | 24-04-2014 |
| | | US 2015209142 A1 | 30-07-2015 |
| | | US 2017027693 A1 | 02-02-2017 |
| | | US 2017065414 A1 | 09-03-2017 |
| | | WO 2010042950 A2 | 15-04-2010 |
| US 2012123515 A1 | 17-05-2012 | KEINE | |
| US 2007239254 A1 | 11-10-2007 | KEINE | |

EPO FORM P0461

Für nähere Einzelheiten zu diesem Anhang : siehe Amtsblatt des Europäischen Patentamts, Nr.12/82